# EUROPEAN PATENT APPLICATION

(11) **EP 4 349 852 A1**
(43) Date of publication of application: **10.04.2024**
(21) Application number: 22810641.5
(22) Date of filing: 27.05.2022
(51) Int. Cl.: C07K 14/005, A61K 38/17, A61K 48/00, C12N 7/00, A61P 27/02

(54) **RECOMBINANT ADENO-ASSOCIATED VIRUS HAVING VARIANT CAPSID, AND APPLICATION THEREOF**

(30) Priority: 28.05.2021 CN 202110594986
(71) Applicant: Shanghai Regenelead Therapies Co., Ltd., Shanghai 201206 (CN)
(72) Inventor: ZHANG, Wentao, Lianyungang, Jiangsu 222047 (CN); LIAO, Cheng, Shanghai 201210 (CN); NING, Wei, Lianyungang, Jiangsu 222047 (CN)
(74) Representative: Dragotti & Associati S.R.L.
(86) International application number: PCT/CN2022/095422
(87) International publication number: WO 2022/247917

(57) **Abstract**

Provided are a recombinant adeno-associated virus (rAAV) having a variant capsid, and an application thereof. Specifically, provided are a variant AAV capsid protein, an rAAV viral particle comprising the variant AAV capsid protein, and an application thereof in delivering a gene product to a cell (e.g., a retinal cell).

## Description

The present application claims priority to Chinese Patent Application No. 202110594986.X filed on May 28, 2021.

### TECHNICAL FIELD

The present disclosure relates to the technical field of recombinant adeno-associated viruses (rAAVs), and in particular to an rAAV virion having a variant capsid and use thereof in the delivery of a gene product to a target cell (e.g., a retinal cell).

### BACKGROUND

Adeno-associated viruses (AAVs), which are small (25 nm), non-enveloped, single-stranded DNA viruses whose nucleic acids are wrapped in icosahedral capsids (caps), belong to the genus Dependovirus of the family Parvoviridae. An AAV contains two open reading frames, rep and cap. Rep encodes four proteins essential for genome replication (Rep78, Rep68, Rep52, and Rep40). Cap encodes three structural proteins required for viral capsid assembly (VP1-3).

As vectors, AAVs have great potential for the treatment of hereditary diseases and genetic diseases and are useful for gene supplementation therapy (also referred to as gene enhancement therapy). AAVs recover or restore the lost or dysregulated gene function resulting from mutations by supplying the missing gene function, thereby restoring the biological function of the target cell to a normal physiological state. The performance of AAV-based vectors in preclinical disease models and in human clinical trials has shown prospects for its application to the treatment of several diseases. For example, a gene can be efficiently transferred into retinal cells and constantly expressed for a long time (Boye et al. Mol Ther.2013 Mar;21(3):509-19. Trapani et al. Prog Retin Eye Res. 2014 Nov;43:108-28). At present, the AAV vectors for clinical use have been engineered to be latent when no helper virus is present. Their safety and long-term expression of transgenes have been extensively tested in rodent models, non-human primates and several human trials (MacLaren et al. Lancet. 2014 Mar 29; 383(9923):1129-37; Maguire et al. N Engl J Med. 2008 May 22;358(21):2240-8; Simonelli et al. Mol Ther. 2010 Mar;18(3):643-50; Nathwani et al. N Engl J Med. 2014 Nov 20;371(21):1994-2004).

AAV capsid proteins occur naturally in different compositions and structures. Different capsids differ in tissue tropism. Multiple homologous primate AAV serotypes and non-human primate AAV serotypes have been identified, and different engineered AAV variants (also referred to as AAV serotypes) have been developed. Although AAV vectors exhibit certain degrees of spread and infection in different organisms and tissues, topical injection into foci is used in most clinical trials, particularly in applications to ocular gene therapy. At present, the major ocular clinical embodiment is subretinal injection (i.e., cavity formation after liquid injection between RPE and photoreceptor cells) and intravitreal injection. Subretinal injection brings AAVs into full contact with RPE and photoreceptor cells, achieving a relatively good local infection effect. However, it involves a relatively high risk relative to injection operations and easily leads to retinal detachment. In intravitreal injection, the AAV formulation will first be evenly distributed in the vitreous humor and then spread and infect the retina layer by layer. Due to the compact structure of the retinal layer and the complex cell population, among natural serotypes, AAV8 and AAV2, which have relatively great abilities to infect eyes, have relatively poor effects when intravitreal injection is adopted, while some engineered capsids show relatively great infection abilities when intravitreal injection is carried out (such as AAVDJ and AAV2.7M8).

There remains a need in the art for novel AAV variants. The present disclosure provides such recombinant adeno-associated virus (rAAV) virions with capsid proteins of new structures, which have high infectivity for retinal tissue and high expression efficiency for heterologous genes, thereby providing a more potential approach to clinical therapy.

### SUMMARY

The present disclosure provides a variant AAV capsid protein and a gene product that can be carried by same, an rAAV virion comprising the capsid protein, a pharmaceutical composition, a method for using the rAAV virion to infect cells (e.g., retinal cells), treat and prevent diseases (e.g., eye diseases), and pharmaceutical use.

### Variant Adeno-Associated Virus (AAV) Capsid Protein

The present disclosure provides an adeno-associated virus (AAV) capsid protein comprising an inserted polypeptide relative to a parental AAV capsid protein, wherein the inserted polypeptide comprises a polypeptide selected from any one of 1)-6) or any combination thereof:
1) LAETTRP (SEQ ID NO: 11) or a polypeptide consisting of SEQ ID NO: 11;
2) LGDTTRP (SEQ ID NO: 12) or a polypeptide consisting of SEQ ID NO: 12;
3) LGETTRN (SEQ ID NO: 13) or a polypeptide consisting of SEQ ID NO: 13;
4) KADTTKN (SEQ ID NO: 14) or a polypeptide consisting of SEQ ID NO: 14;
5) KDDTTRN (SEQ ID NO: 15) or a polypeptide consisting of SEQ ID NO: 15; and
6) LADTTKN (SEQ ID NO: 16) or a polypeptide consisting of SEQ ID NO: 16.

The present disclosure provides an adeno-associated virus (AAV) capsid protein comprising an inserted polypeptide relative to a parental AAV capsid protein, the polypeptide comprising X₁X₂X₃TTX₄X₅ (SEQ ID NO: 35) or consisting of SEQ ID NO: 35, wherein X₁ is selected from the group consisting of L and K, X₂ is selected from the group consisting of G, D and A, X₃ is selected from the group consisting of D and E, X₄ is selected from the group consisting of R and K, and X₅ is selected from the group consisting of P and N.

In some embodiments, the polypeptide set forth in the aforementioned 1)-6) or SEQ ID NO: 35 has 1-4 spacer amino acids at the amino-terminus and/or carboxy-terminus thereof (Y₁-Y₄). In some specific embodiments, the spacer amino acids include, but are not limited to, A, L, G, S and T.

The present disclosure provides an adeno-associated virus (AAV) capsid protein comprising an inserted polypeptide relative to a parental AAV capsid protein, the polypeptide comprising Y₁Y₂X₁X₂X₃TTX₄X₅Y₃Y₄ (SEQ ID NO: 36) or consisting of SEQ ID NO: 36, wherein X₁ is selected from the group consisting of L and K, X₂ is selected from the group consisting of G, D and A, X₃ is selected from the group consisting of D and E, X₄ is selected from the group consisting of R and K, and X₅ is selected from the group consisting of P and N; Y₁, Y₂, Y₃ and Y₄ may be independently present or absent, and Y₁, Y₂, Y₃ and Y₄ may be independently selected from the group consisting of A, L, G, S and T. For example, Y₁ is L, Y₂ is A, Y₃ is A, and Y₄ is absent.

The present disclosure provides an adeno-associated virus (AAV) capsid protein comprising an inserted polypeptide relative to a parental AAV capsid protein, wherein the inserted polypeptide is selected from the polypeptide shown in any one of 1-1) to 1-6) or any combination thereof:
1-1) a polypeptide comprising LALAETTRPA (SEQ ID NO: 17) or consisting of SEQ ID NO: 17;
2-1) a polypeptide comprising LALGDTTRPA (SEQ ID NO: 18) or consisting of SEQ ID NO: 18;
3-1) a polypeptide comprising LALGETTRNA (SEQ ID NO: 19) or consisting of SEQ ID NO: 19;
4-1) a polypeptide comprising LAKADTTKNA (SEQ ID NO: 20) or consisting of SEQ ID NO: 20;
5-1) a polypeptide comprising LAKDDTTRNA (SEQ ID NO: 21) or consisting of SEQ ID NO: 21; and
6-1) a polypeptide comprising LALADTTKNA (SEQ ID NO: 22) or consisting of SEQ ID NO: 22.

In some embodiments, the AAV capsid protein described above is an AAV2 capsid protein or an AAV9 capsid protein.

In some embodiments, the polypeptide of the aforementioned 1) to 6), 1-1) to 6-1), SEQ ID NO: 35 or 36 is located within a GH loop or IV loop (loop domain IV) of the parental AAV capsid protein, for example, in a solvent accessible portion of the GH loop or IV loop of the AAV capsid protein (see van Vliet et al. (2006) Mol. Ther. 14:809; Padron et al. (2005) J. Virol. 79:5047; and Shen et al. (2007) Mol. Ther. 15:1955). "Parental AAV capsid protein" refers to a capsid protein of the same AAV serotype without an inserted polypeptide (including wild-type AAV serotypes or variant capsid proteins thereof, e.g., the AAV2 capsid protein set forth in SEQ ID NO: 1 or the AAV9 capsid protein set forth in SEQ ID NO: 37 of the present disclosure; the AAV2 capsid protein may or may not have the V708I mutation).

In some embodiments, the AAV is selected from the group consisting of AAV1, AAV2, AAV3, AAV4, AAV5, AAV6, AAV7, AAV8, AAV9 and AAV 10 (including AAVrh10).

In some embodiments, the aforementioned inserted polypeptide is located within amino acid residue positions 411 to 650, or 432 to 640, or 570 to 671, or 570 to 614, or 570 to 610, or 580 to 600, or 570 to 575, or 575 to 580, or 580 to 585, or 585 to 590, or 590 to 600, or 600 to 614, of the parental AAV capsid protein; for example, within amino acid residue positions 570 to 611 of a parental AAV2 capsid protein, within amino acid residue positions 571 to 612 of a parental AAV1 capsid protein, within amino acid residue positions 560 to 601 of a parental AAV5 capsid protein, within amino acid residue positions 571 to 612 of a parental AAV6 capsid protein, within amino acid residue positions 572 to 613 of a parental AAV7 capsid protein, within amino acid residue positions 573 to 614 of a parental AAV8 capsid protein, within amino acid residue positions 571 to 612 of a parental AAV9 capsid protein, or within amino acid residue positions 573 to 614 of a parental AAV 10 (including AAVrh10) capsid protein.

In some embodiments, the aforementioned inserted polypeptide is located between amino acid residue positions 587 and 588 of a parental AAV2 capsid protein, or between amino acid residue positions 588 and 589 of a parental AAV9 capsid protein, or at a corresponding position of capsid proteins of other parental AAV serotypes. In some embodiments, the inserted polypeptide is located between amino acid residue positions 587 and 588 of a parental AAV2 capsid protein, or between amino acid residue positions 588 and 589 of a parental AAV9 capsid protein, or at a corresponding position of capsid proteins of other parental AAV serotypes. Other serotypes are, for example, selected from the group consisting of AAV1, AAV3, AAV4, AAV5, AAV6, AAV7, AAV8 and AAV10 (including AAVrh10). Sequences in different AAV serotypes corresponding to amino acids 570-611 of the capsid protein VP1 of AAV2 are well known in the art (see, e.g., FIG. 6 of WO2012145601A, and AAV1 of GenBank accession number NP_049542; AAVS of AAD13756; AAV6 of AAB95459; AAV7 of YP_077178; AAV8 of YP_077180; AAV9 of AAS99264 and AAV10 of AAT46337). In some embodiments, the inserted polypeptide is located between amino acid residue positions 590 and 591 of a parental AAV1 capsid protein, between amino acid residue positions 575 and 576 of a parental AAV5 capsid protein, between amino acid residue positions 590 and 591 of a parental AAV6 capsid protein, between amino acid residue positions 589 and 590 of a parental AAV7 capsid protein, between amino acid residue positions 590 and 591 of a parental AAV8 capsid protein, or between amino acid residue positions 588 and 589 of a parental AAV10 (including AAVrh10) capsid protein.

The numbering of amino acid residues of the present disclosure is natural numbering starting from the N-terminus relative to the encoded amino acid sequence of VP1 of the AAV capsid protein. For example, "the inserted polypeptide is located between amino acid residue positions 587 and 588 of an AAV2 capsid protein" means that the polypeptide is located between positions 587 and 588 of the encoded amino acid sequence of VP1 of the AAV2 capsid protein; correspondingly, the polypeptide is located between positions 450 and 451 of the encoded amino acid sequence of VP2, and between positions 385 and 386 of the encoded amino acid sequence of VP3.

In some embodiments, the aforementioned inserted polypeptide is located between amino acid residue positions 450 and 460 of the parental AAV capsid protein; for example, the polypeptide precedes or follows amino acid residue position 453 of parental AAV2, amino acid residue position 454 of parental AAV1, amino acid residue position 454 of parental AAV6, amino acid residue position 456 of parental AAV7, amino acid residue position 456 of parental AAV8, amino acid residue position 454 of parental AAV9, or amino acid residue position 456 of parental AAV10 (including AAVrh10). The amino acid residues of capsid proteins of different AAV serotypes and correspondences thereof set forth in FIG. 17 of WO2012145601A are incorporated herein by reference in their entirety.

In some embodiments, the present disclosure provides a variant AAV capsid protein comprising the polypeptide of the aforementioned 1) to 6), 1-1) to 6-1), SEQ ID NO: 35 or 36.

In some embodiments, the aforementioned variant AAV capsid protein of the present disclosure further comprises one or more amino acid residue point mutations (including substitutions, deletions and/or additions).

In some embodiments, the amino acid residue point mutation(s) is/are located at one of positions 1, 15, 34, 57, 66, 81, 101, 109, 144, 164, 176, 188, 196, 226, 236, 240, 250, 312, 363, 368, 449, 456, 463, 472,484, 524, 535, 551, 593, 698, 708, 719, 721 and 735, or any combination thereof.

In some embodiments, the amino acid residue point mutation(s) (substitution(s)) is/are selected from one of IL, 15P, 34A, 57D, 66K, 81Q, 101R, 109T, 144K or M, 164K, 176P, 188I, 196Y, 226E, 236V, 240T, 250S, 312K, 363L, 368H, 449D, 456K, 463Y, 472N, 484C, 524T, 535S, 551S, 593E, 698V, 708I, 719M, 721L and 735Q, or any combination thereof, e.g., one or more of 312K, 449D, 472N, 551S, 698V, 735Q, 273F, 444F, 500F, 730F and 708I, e.g., 708I.

In some embodiments, the amino acid residue point mutation(s) (substitution(s)) is/are selected from one of M1L, LISP, P34A, N57D, N66K, R81Q, Q101R, S109T, R144K, R144M, Q164K, T176P, L188I, S196Y, G226E, G236V, I240T, P250S, N312K, P363L, D368H, N449D, T456K, S463Y, D472N, R484C, A524T, P535S, N551S, A593E, I698V, V708I, V719M, S721L, L735Q, Y273F, Y444F, Y500F and Y730F, or any combination thereof, e.g., one or more of N312K, N449D, D472N, NSSI S, I698V, L735Q, Y273F, Y444F, Y500F, Y730F and V7081.

In some specific embodiments, the amino acid residue point mutation(s) (substitution(s)) is/are 708I and/or 449D, or V708I and/or N449D. In some specific embodiments, the amino acid residue mutation(s) (substitution(s)) is/are 273F, 444F, 500F and/or 730F, or Y273F, Y444F, Y500F and/or Y730F.

The above point mutations are relative to the corresponding positions of the corresponding parental AAV capsid protein, for example, relative to the corresponding positions of a parental AAV2 capsid protein.

The mutations in the capsid proteins of WO2012145601A, WO2017197355A and WO2018022905A are incorporated in the present disclosure by reference in their entirety. In some embodiments, the AAV capsid of the present disclosure is a chimeric capsid. For example, the capsid comprises a portion of a first AAV serotype AAV capsid and a portion of a second serotype AAV capsid, the serotype including, but not limited to, AAV1, AAV2, AAV3, AAV4, AAV5, AAV6, AAV7, AAV8, AAV9 and AAV10 (including AAVrh10). For example, the AAV capsid may be AAV2G9, which comprises sequences from AAV2 and AAV9. The sequence of AAV2G9 in US20160017005 is incorporated herein by reference in its entirety.

In some embodiments, the variant AAV capsid protein of the present disclosure is isolated and/or purified.

In some embodiments, the present disclosure provides a variant AAV2 capsid protein comprising a polypeptide in a GH loop or IV loop (loop domain IV) of the capsid protein, relative to a corresponding parental AAV2 capsid protein (e.g., as set forth in SEQ ID NO: 1), wherein the polypeptide is selected from the group consisting of the aforementioned 1) to 6), 1-1) to 6-1), SEQ ID NOs: 35 and 36. In some specific embodiments, the polypeptide comprises or is SEQ ID NO: 12 or 18. In some specific embodiments, the polypeptide is located between amino acid residue positions 587 and 588 of VP1 of the parental AAV2 capsid protein.

In some embodiments, the present disclosure provides a variant AAV9 capsid protein comprising a polypeptide in a GH loop or IV loop (loop domain IV) of the capsid protein, relative to a corresponding parental AAV9 capsid protein (e.g., as set forth in SEQ ID NO: 37), wherein the polypeptide is selected from the group consisting of the aforementioned 1) to 6), 1-1) to 6-1), SEQ ID NOs: 35 and 36. In some specific embodiments, the polypeptide comprises or is SEQ ID NO: 12 or 18. In some specific embodiments, the polypeptide is located between amino acid residue positions 588 and 589 of VP1 of the parental AAV9 capsid protein.

In some embodiments, the present disclosure provides a variant AAV capsid protein having an amino acid sequence that is set forth in any one of SEQ ID NOs: 3-9 and 23-27 or has at least 85%, at least 90%, at least 95%, at least 96%, at least 97%, at least 98% or at least 99% sequence identity thereto.

In some embodiments, the variant AAV capsid protein provided by the present disclosure has the following properties:
(a) increased infectivity for eye tissue (such as retinal cells), particularly increased infectivity for retinal nerve cell layers, as compared to the infectivity of an AAV virion comprising a corresponding parental AAV capsid protein for eye tissue (such as retinal cells);
(b) altered cellular tropism, as compared to the tropism of an AAV virion comprising a corresponding parental AAV capsid protein;
(c) increased ability to bind and/or cross an internal limiting membrane (ILM), as compared to an AAV virion comprising a corresponding parental AAV capsid protein; and/or
(d) increased expression of the gene product wrapped or carried therein in eye tissue (such as retinal tissue, aqueous humor or vitreous humor), as compared to an AAV virion comprising a corresponding parental AAV capsid protein.

### Recombinant Adeno-Associated Virus (rAAV) Virion

The present disclosure provides a recombinant adeno-associated virus (rAAV) virion comprising:
(a) any of the aforementioned variant AAV capsid proteins of the present disclosure; alternatively, comprising
(b) a heterologous polynucleotide.

In some embodiments, the heterologous polynucleotide comprises a polynucleotide that expresses or encodes a gene product. In some embodiments, the gene product is heterologous relative to AAV. In some embodiments, the gene product is heterologous or endogenous relative to the target cell. In some embodiments, the gene product is one or more (e.g., 2, 3 or 4) gene products.

In some embodiments, the heterologous polynucleotide comprises a regulatory sequence that regulates the expression or encoding of the gene product.

### Regarding the gene product:

In some embodiments, the gene product is therapeutic or prophylactic against a disease or disorder.

In some embodiments, the gene product is selected from the group consisting of an interfering RNA (RNAi), an aptamer and a polypeptide.

In some specific embodiments, the gene product is an RNAi, e.g., an RNAi that decreases or reduces the level of an apoptotic or angiogenic factor in a cell. For example, the RNAi may be a shRNA or siRNA that reduces the levels of induced or pro-apoptotic gene products in cells. The pro-apoptotic gene products include, for example, Bax, Bid, Bak and Bad gene products (see US7,846,730, which is incorporated herein by reference in its entirety). As another example, the RNAi may be directed against angiogenic products, such as VEGF (e.g., Cand5, see US2011/0143400 and US2008/0188437, which are incorporated herein by reference in their entirety), VEGFR1 (e.g., Sima-027, see Kaiser et al. (2010) Am. J. Ophthalmol. 150:33; and Shen et al. (2006) Gene Ther. 13:225, which are incorporated herein by reference in their entirety), or VEGFR2 (e.g., Kou et al. (2005) Biochem. 44:15064, which is incorporated herein by reference in its entirety).

In some specific embodiments, the gene product is an aptamer, such as a specific aptamer for VEGF (e.g., 5'-cgcaaucagugaaugcuuauacauccg-3', see Ng et al. (2006) Nat. Rev. Drug Discovery 5:123, and Lee et al. (2005) Proc. Natl. Acad. Sci. USA 102:18902, which are incorporated herein by reference in their entirety), or a specific aptamer for PDGF (e.g., E10030, see Ni and Hui (2009) Ophthalmologica 223:401, and Akiyama et al. (2006) J. Cell Physiol. 207:407, which are incorporated herein by reference in their entirety).

In some specific embodiments, the gene product is a polypeptide.

In some specific embodiments, the gene product is a neuroprotective polypeptide, an anti-angiogenic polypeptide, or a polypeptide that enhances retinal cell function.

In some specific embodiments, the polypeptide can enhance the function of retinal cells, for example, enhance the function of rod or cone photoreceptor cells, retinal ganglion cells, Muller cells, bipolar cells, amacrine cells, horizontal cells, or retinal pigment epithelial cells.

In some specific embodiments, the polypeptide includes or is selected from the group consisting of: neuroprotective polypeptides (such as GDNF, CNTF, NT4, NGF and NTN); anti-angiogenic polypeptides (such as soluble vascular endothelial growth factor (VEGF) receptors, anti-VEGF antibodies or antigen-binding fragments thereof, endostatin, tumstatin, angiostatin, soluble Fit polypeptide and fusion proteins thereof with an Fc region (see Lai et al. (2005) Mol. Ther. 12:659, and Pechan et al. (2009) Gene Ther. 16:10), pigment epithelium derived factor (PEDF), soluble Tie-2 receptor, and the like); tissue inhibitor of metalloproteinase 3 (TIMP-3); light-responsive opsins (such as rhodopsin); anti-apoptotic polypeptides (such as Bcl-2 and Bcl-Xl), and the like.

In some specific embodiments, the polypeptide includes, but is not limited to: epidermal growth factor, rhodopsin and X-linked inhibitor of apoptosis protein.

In some specific embodiments, the polypeptide includes, but is not limited to: retinoschisin, retinitis pigmentosa GTPase regulator (RGPR) interacting protein-1 (GenBank Accession NOs. Q96KN7, Q9EPQ2 and Q9GLM3), peripherin-2 (Prph2) (GenBank Accession NO. NP_000313), and retinal pigment epithelium specific protein (RPE65) (GenBank Accession NO. AAC39660).

In some specific embodiments, the polypeptide includes, but is not limited to: polypeptides that induce choroideremia when damaged or deleted, such as CHM (choroidermia (Rab escort protein 1)) (Donnelly et al. (1994) Hum. Mol. Genet. 3:1017); polypeptides that induce Leber's congenital amaurosis when damaged or deleted and polypeptides of retinitis pigmentosa, such as crumbs homolog 1 (CRB1) (GenBank Accession NO. CAM23328); and polypeptides that induce achromatopsia when damaged or deleted, such as rod photo-sensitive cGMP-gated channel subunit α (CNGA3) (GenBank Accession NO. NP_001289), rod photo-sensitive cGMP-gated channel β subunit (CNGB3), guanine nucleotide-binding protein (G protein), α transduction active polypeptide 2 (GNAT2) (ACHM4), ACHM5, L-opsin, M-opsin, and S-opsin.

In some specific embodiments, the gene product provides a site-specific endonuclease for site-specific knock-down of gene function, e.g., wherein the endonuclease knocks out an allele associated with a retinal disease. For example, where a dominant allele encodes a defective copy of a gene that, when belonging to a wild-type, is a retinal structural protein and/or provides normal retinal function, a site-specific endonuclease can be targeted to the defective allele and knock out the defective allele. Examples of the site-specific endonuclease are zinc finger nucleases (ZFNs), and transcription activator-like effector nucleases (TALENs), wherein such site-specific endonucleases are non-naturally occurring and are modified to target a specific gene.

Furthermore, the sequences and sources of the aforementioned gene products in WO2012145601A, WO2017197355A and WO2018022905A are incorporated in the present disclosure by reference in their entirety.

In some specific embodiments, the gene product of the present disclosure is an anti-angiogenic agent, including anti-angiogenic polypeptides, e.g., anti-VEGF antibodies or antigen-binding fragments thereof; or e.g., VEGF antagonists (e.g., VEGF-A, B and C antagonists) or PDGF antagonists.

In some specific embodiments, VEGF antagonists include, but are not limited to, ranibizumab, bevacizumab, aflibercept, KH902 VEGF receptor-Fc fusion protein, 2C3 antibody, ORA102, pegaptanib, bevasiranib, SIRNA-027, decursin, decursinol, picropodophyllin, guggulsterone, PLG101, eicosanoid LXA4, PTK787, pazopanib, axitinib, CDDO-Me, CDDO-Imm, shikonin, β-hydroxyisovalerylshikonin, or ganglioside GM3, DC101 antibody, Mab25 antibody, Mab73 antibody, 4A5 antibody, 4E10 antibody, SF12 antibody, VA01 antibody, BL2 antibody, VEGF-related proteins, sFLT0l, sFLT02, peptide B3, TG100801, sorafenib, sunitnab, G6-31 antibody, or pharmaceutically acceptable salts thereof. The information about VEGF antagonists in WO2018160686A is incorporated herein by reference in its entirety.

For the sequence of ranibizumab (Lucentis^{®}), see US7,060,269 (FIG. 1 therein). For the sequence of bevacizumab (Avastin^{®}), see US6,054,297 (FIG. 1 therein). For the sequence of aflibercept (Eyelea^{®}), see Do et al. (Br J Ophthalmol. 2009,93:144-9). These are incorporated herein by reference in their entirety.

In some embodiments, the VEGF antagonist comprises or is a naturally occurring protein sFlt-1, or a functional fragment thereof (e.g., sFlt-1 domain 2; for the sequence of sFlt-1, see US5,861,484; for the sequence of sFlt-1 domain 2, see US2013/0323302; these are incorporated herein by reference in their entirety).

In some embodiments, the VEGF antagonist is a VEGF-binding fusion protein. The sequence of the VEGF-binding fusion protein in US7,635,474 is incorporated herein by reference in its entirety.

In some specific embodiments, the amino acid sequence of aflibercept is set forth in SEQ ID NO: 38, and a polynucleotide sequence encoding SEQ ID NO: 38, e.g., a codon-optimized polynucleotide sequence set forth in any one of SEQ ID NOs: 39-41, is provided.

### Regarding the regulatory sequence:

In some embodiments, the aforementioned polynucleotide comprises the polynucleotide (regulatory sequence) of any one of (a)-(h) below or any combination thereof:
(a) a 5' inverted terminal repeat (5' ITR) and/or a 3' inverted terminal repeat (3' ITR);
(b) a 5' untranslated region (5' UTR) and/or a 3' untranslated region (3' UTR);
(c) a promoter;
(d) an enhancer;
(e) an intron;
(f) a post-transcriptional regulatory element;
(g) a polyadenylation signal (polyA); and
(h) a Kozak sequence.

In some embodiments, AAV ITRs need not have a wild-type nucleotide sequence and can be altered by insertion, deletion or substitution of nucleotides, or AAV ITRs can be obtained from any one of several AAV serotypes, such as AAV1, AAV2, AAV3, AAV4, AAV5, AAV6, AAV7, AAV8, AAV9 and AAV10. In some specific embodiments, the 5' ITR and 3' ITR are the 5' ITR and 3' ITR of AAV2.

In some embodiments, the nucleotide sequence encoding a gene product is operably linked to a tissue-specific or cell-type specific regulatory element, e.g., a photoreceptor-specific regulatory element (e.g., a photoreceptor-specific promoter), or e.g., a regulatory element that confers selective expression of the operably linked gene in a photoreceptor cell.

In some specific embodiments, any combination of (a)-(h) can fulfill the function of allowing expression of a gene product (e.g., an anti-angiogenic agent (or anti-angiogenic polypeptide), or e.g., an anti-VEGF antibody or an antigen-binding fragment thereof, or aflibercept), e.g., the function of allowing expression in eye tissue (e.g., the aqueous humor or retinal tissue) of a subject.

In some specific embodiments, the polynucleotide of any one of (a)-(h), or any combination thereof, is operably linked to the aforementioned polynucleotide encoding a gene product.

In some specific embodiments, the 5' ITR and/or 3' ITR are/is derived from AAV1, AAV2, AAV3, AAV4, AAV5, AAV6, AAV7, AAV8, AAV9, AAV9.47, AAV9(hu14), AAV10, AAV11, AAV12, AAVrh8, AAVrh10, AAV-DJ or AAV-DJ8; for example, from AAV2 or AAV9.

In some specific embodiments, the 5' UTR and/or 3' UTR are/is derived from hemopexin (HPX), hemoglobin subunit beta (HBB), HSPB1, CCL13, or Xenopus globin. For example, the sequence of the 5' UTR is set forth in SEQ ID NO: 30, and the sequence of the 3' UTR is set forth in SEQ ID NO: 32.

In some specific embodiments, the promoter may be a constitutive promoter or an inducible promoter.

In some specific embodiments, the promoter is selected from the group consisting of a cytomegalovirus (CMV) promoter, a Rous sarcoma virus (RSV) promoter, a UB6 promoter, a chicken β-actin promoter, a CAG promoter, an RPE65 promoter, a CBh promoter, an EFS promoter, an EF1 (e.g., EF-1α) promoter, a PGK promoter, an SV40 promoter, a Ubi promoter, an opsin promoter, and any combination thereof. The opsin promoter includes, but is not limited to, the rhodopsin promoter, the rhodopsin kinase promoter (Young et al. (2003) Ophthalmol. Vis. Sci. 44:4076), the β phosphodiesterase gene promoter (Nicoud et al. (2007) J. Gene Med. 9:1015), the retinitis pigmentosa gene promoter (Nicoud et al. (2007) supra), and the interphotoreceptor retinoid-binding protein (IRBP) gene promoter (Yokoyama et al. (1992) Exp Eye Res. 55:225). For example, the promoter is a CMV promoter, the sequence of which is, for example, set forth in SEQ ID NO: 29.

In some specific embodiments, the enhancer is selected from the group consisting of Ubi, CMV, RSV, the IRBP gene enhancer (Nicoud et al. (2007) J. Gene Med. 9: 1015), and any combination thereof. For example, the enhancer is a CMV enhancer, the sequence of which is, for example, set forth in SEQ ID NO: 28.

In some specific embodiments, the intron is selected from the group consisting of MVM, SV40, β Globin, EF1 (e.g., EF-1α), a hybrid intron, and any combination thereof.

In some specific embodiments, the polyA is selected from the group consisting of PA75 polyA, SV40 polyA, hGH polyA, BGH polyA, rbGlob polyA, and any combination thereof. For example, the polyA is SV40 polyA, the sequence of which is, for example, set forth in SEQ ID NO: 34.

In some specific embodiments, the post-transcriptional regulatory element is selected from the group consisting of WPRE, HPRE, and a combination thereof. For example, the post-transcriptional regulatory element is WPRE, the sequence of which is, for example, set forth in SEQ ID NO: 33.

In some embodiments, the aforementioned polynucleotide comprises the polynucleotide (regulatory sequence) of any one of (a)-(g) below or any combination thereof:
(a) a 5' ITR and/or a 3' ITR derived from AAV2;
(b) a 5' UTR and/or a 3' UTR derived from Xenopus globin;
(c) a CMV promoter;
(d) a CMV enhancer;
(e) WPRE;
(f) a Kozak sequence; and
(g) SV40 polyA.

Illustratively, the sequence of the polynucleotide from the 5' end to the 3' end is (alternative regulatory sequences in parentheses):
5' ITR-enhancer-promoter-5' UTR-(Kozak sequence)-gene product-3' UTR-(WPRE)-polyA-3' ITR, or
enhancer-promoter-5' UTR-(Kozak sequence)-gene product-3' UTR-(WPRE)-polyA; promoter-(5' UTR)-(Kozak sequence)-gene product-(3' UTR)-(WPRE)-polyA.

### Regarding the effect of the rAAV virion:

In some embodiments, the rAAV virion is infectious; in some other embodiments, the rAAV virion is not infectious.

In some embodiments, the aforementioned rAAV virion of the present disclosure shows an at least 2-fold, at least 3-fold, at least 4-fold, at least 5-fold, at least 10-fold, at least 15-fold, at least 20-fold, at least 25-fold, at least 50-fold, or more than 50-fold increase in infectivity for a cell, as compared to an AAV virion comprising a corresponding parental AAV capsid protein, wherein the cell is selected from the group consisting of:
1) a retinal cell;
2) a photoreceptor cell;
3) an RPE cell;
4) a bipolar cell;
5) an amacrine cell; and/or
6) a horizontal cell.

In some embodiments, the aforementioned rAAV virion of the present disclosure shows an at least 2-fold, at least 3-fold, at least 4-fold, at least 5-fold, at least 10-fold, at least 15-fold, at least 20-fold, at least 25-fold, at least 50-fold, or more than 50-fold increase in the ability to cross an internal limiting membrane (ILM), as compared to an AAV virion comprising a corresponding parental AAV capsid protein.

In some embodiments, the aforementioned rAAV virion of the present disclosure selectively infects retinal cells. For example, the aforementioned rAAV virion of the present disclosure infects retinal cells with 2 times, 3 times, 4 times, 5 times, 10 times, 15 times, 20 times, 25 times, 50 times or more than 50 times higher specificity than it infects non-retinal cells (e.g., non-eye tissue cells).

In some embodiments, the aforementioned rAAV virion of the present disclosure (e.g., the rAAV virion in which the capsid protein comprises seq2) shows a 2 times, 3 times, 4 times, 5 times, 10 times, 15 times, 20 times, 25 times, 50 times or more than 50 times higher expression level of a gene product (such as aflibercept) in whole-eye tissue than the rAAV virion comprising the AAV2.7m8 capsid protein.

In some embodiments, the aforementioned rAAV virion of the present disclosure (e.g., the rAAV virion in which the capsid protein comprises seq2) shows a 2 times, 3 times, 4 times, 5 times, 10 times, 15 times, 20 times, 25 times, 50 times or more than 50 times higher expression level of a gene product (such as aflibercept) in the aqueous humor than the rAAV virion comprising the AAV2.7m8 capsid protein.

In some embodiments, the aforementioned rAAV virion of the present disclosure (e.g., the rAAV virion in which the capsid protein comprises seq2) shows a 2 times, 3 times, 4 times, 5 times, 10 times, 15 times, 20 times, 25 times, 50 times or more than 50 times higher expression level of a gene product (such as aflibercept) in the vitreous humor than the rAAV virion comprising the AAV2.7m8 capsid protein.

In some embodiments, the aforementioned rAAV virion of the present disclosure (e.g., the rAAV virion in which the capsid protein comprises seq2) shows a 2 times, 3 times, 4 times, 5 times, 10 times, 15 times, 20 times, 25 times, 50 times or more than 50 times higher expression level of a gene product (such as aflibercept) in retinal tissue than the rAAV virion comprising the AAV2.7m8 capsid protein.

In some embodiments, the route of administering the rAAV virion described above is intravitreal injection.

### Polynucleotide and (Expression) Vector

The present disclosure provides an aflibercept-encoding polynucleotide, and the amino acid sequence it encodes is set forth in SEQ ID NO: 38.

In some embodiments, the present disclosure provides a codon-optimized polynucleotide encoding aflibercept, which is set forth in any one of SEQ ID NOs: 39-41 or has at least 80%, 85%, 90%, 95%, 96%, 97%, 98% or 99% sequence identity thereto.

The present disclosure provides a polynucleotide encoding any of the aforementioned gene products of the present disclosure.

The present disclosure provides a polynucleotide encoding any of the aforementioned AAV capsid proteins of the present disclosure.

In some embodiments, the polynucleotide described above may be an RNA, a DNA or a cDNA.

In some embodiments, the polynucleotide described above is an isolated polynucleotide. The polynucleotide of the present disclosure may be in the form of, may be present in, and/or may be part of a vector, such as a plasmid, cosmid, YAC, or viral vector. The vector may be, for example, an expression vector, i.e., a vector that can provide expression of the polynucleotide *in vitro* and/or *in vivo* (i.e., in a suitable host cell, host organism, and/or expression system). The expression vector generally comprises at least one of the polynucleotides of the present disclosure, which is operably linked to one or more suitable expression regulatory elements (e.g., promoters, enhancers, terminators, and the like). The polynucleotide of the present disclosure can be prepared or obtained by known means (e.g., by automatic DNA synthesis and/or recombinant DNA techniques), and/or can be isolated from a suitable natural source.

The present disclosure provides a vector comprising:
(a) an isolated polynucleotide encoding any of the aforementioned variant AAV capsid proteins of the present disclosure (which is set forth in any one of SEQ ID NOs: 3-9 and 23-27 or has at least 90% or 95% sequence identity thereto);
(b) an isolated polynucleotide encoding any of the aforementioned variant AAV capsid proteins of the present disclosure (which is set forth in any one of SEQ ID NOs: 3-9 and 23-27 or has at least 90% or 95% sequence identity thereto); and/or the aforementioned heterologous polynucleotide encoding a gene product (e.g., the polynucleotide encoding aflibercept set forth in SEQ ID NO: 38, or a polynucleotide set forth in any one of SEQ ID NOs: 39-41 or having at least 90% or 95% sequence identity thereto).

In some embodiments, the polynucleotide encoding the variant AAV capsid protein and the heterologous polynucleotide encoding a gene product in (b) are in different vectors.

### Host Cell

The present disclosure provides a host cell comprising any of the aforementioned polynucleotides or (expression) vectors of the present disclosure.

In some embodiments, the host cell may be an isolated cell, such as a cell from an *in vitro* cell culture. Such cells are useful for producing any of the aforementioned rAAV capsid proteins, gene products or rAAV virions of the present disclosure and hence are also known as producer cells.

In some specific embodiments, the producer cell is a bacterial cell, a fungal cell or a mammalian cell. Exemplary producer cells include, but are not limited to, HeLa, CHO, 293 (including 293T), Vero, NIH 3T3, Huh-7, BHK, PC12, COS (including COS-7), RAT1, HepG2 cells, and the like. Exemplary mammalian cells include, but are not limited to, 293 (293T), COS, HeLa, Vero, 3T3, C3H10T1/2 and CHO cells. Amphibian cells, insect cells, plant cells, and any other cells used in the art for expressing proteins or virions can also be used as producer cells. Exemplary insect cells include, but are not limited to, Spodoptera frugiperda, the Drosophila cell line, or mosquito cell lines, such as the Aedesalbopictus derived cell lines, including but not limited to, Se301, SeIZD2109, SeUCRl, Sf9, Sf900+, Sf21, BTI-TN-5B1-4, MG-1, Tn368, HzAml, Ha2302, Hz2E5, HighFive (Invitrogen, CA, USA), AO38 and BM-N cells.

### Production and Preparation Methods for rAAV Virions

Methods for producing rAAV virions are conventional in the art. The production and preparation methods for rAAV virions in WO200028004, WO200123001, WO2004112727, WO 2005005610, WO2005072364, WO2013123503, WO2015191508 and US20130195801 are incorporated in the present disclosure by reference in their entirety. The rAAV virions can have enhanced delivery efficiency, can be efficiently packaged, and can successfully infect a target cell (e.g., a mammalian or human cell) with high frequency and minimal toxicity.

The present disclosure provides a method for producing and preparing an rAAV virion, comprising packaging any of the aforementioned polynucleotides of the present disclosure into an AAV capsid.

In some embodiments, provided is a method for producing and preparing an rAAV virion, comprising: introducing the aforementioned polynucleotide or (expression) vector thereof of the present disclosure comprising an encoded gene product, a polynucleotide or a (expression) vector thereof encoding any of the aforementioned AAV capsid proteins of the present disclosure, and a helper functional plasmid (e.g., pHelper) into a producer cell (e.g., a 293 cell), packaging and purifying to obtain the rAAV virion.

In some embodiments, provided is a production method for producing and preparing an rAAV virion, comprising:
1) simultaneously co-transfecting a mammalian cell (e.g., a 293 cell) with any of the aforementioned polynucleotides or (expression) vectors of the present disclosure comprising an encoded gene product, a vector expressing the Rep and Cap genes (e.g., pR2C9), and a helper vector (for fulfilling helper function, e.g., pHelper), the vector expressing the Rep and Cap genes comprising a polynucleotide encoding any of the aforementioned AAV capsid proteins of the present disclosure; and
2) harvesting and purifying the rAAV virion of the polynucleotide comprising an encoded gene product.

In some embodiments, provided is a production system for an rAAV virion, for use in the production of any of the aforementioned rAAV virions of the present disclosure, comprising:
1) a polynucleotide encoding an AAV capsid protein;
2) any of the heterologous polynucleotides or (expression) vectors thereof (e.g., pGOI plasmid) of the present disclosure comprising an encoded gene product; and
3) a helper element, having sufficient AAV rep function and helper function to package the heterologous polynucleotide comprising an encoded gene product in 2) into the AAV capsid.

In some specific embodiments, the sufficient AAV rep function and helper function are provided by a packaging cell or the three plasmids pHelper, pR2C9 and pGOI, wherein the packaging cell can comprise the three plasmids pHelper, pR2C9 and pGOI.

In some embodiments, the Rep genes encode the non-structural proteins that regulate functions such as the replication of the AAV genome, and may be selected from the group consisting of Rep78, Rep68, Rep52 and Rep40. Rep78 and Rep68 are generally transcribed from the p5 promoter, while Rep52 and Rep40 are generally transcribed from the p19 promoter. The Cap genes encode the structural proteins VP1, VP2 and/or VP3 that assemble to form the viral capsid. The Cap genes are generally transcribed from the p40 promoter.

In some embodiments, rAAV virions produced by the above AAV production method or production system are provided.

### Pharmaceutical Composition

The present disclosure provides a pharmaceutical composition comprising:
(a) any of the aforementioned AAV capsid proteins or rAAV virions of the present disclosure; and
(b) one or more pharmaceutically acceptable carriers, diluents, excipients or buffers.

The present disclosure provides a pharmaceutical composition comprising:
(a) a prophylactically or therapeutically effective amount of an active ingredient; and
(b) one or more pharmaceutically acceptable carriers, diluents, excipients or buffers.

The active ingredient is, for example, selected from the group consisting of: any of the aforementioned rAAV virions of the present disclosure, and a polynucleotide encoding SEQ ID NOs: 39-41 or a gene product having at least 80%, 85%, 90%, 95%, 96%, 97%, 98% or 99% identity sequence thereto.

In some embodiments, the pharmaceutical composition may comprise 0.01 to 99 weight% of a polynucleotide (e.g., a VEGF inhibitor, or a coding polynucleotide for aflibercept) or rAAV virion in a unit dose. In some specific embodiments, the pharmaceutical composition comprises 0.1 to 10 × 10¹³ copies of a gene product (e.g., a VEGF inhibitor, or aflibercept) in a unit dose. In some specific embodiments, the concentration of the rAAV virion in the pharmaceutical composition is 1 × 10⁸ or more per milliliter, typically no more than 1 × 10¹⁵ per milliliter.

In some embodiments, a cell can be transfected with any of the aforementioned rAAV virions of the present disclosure, and the cell is subsequently transferred to or implanted in a subject.

In some embodiments, the pharmaceutical composition comprises any of the aforementioned rAAV virions of the present disclosure, wherein a polynucleotide is wrapped in the rAAV virion, and the polynucleotide is set forth in any one of SEQ ID NOs: 39-41 or is a sequence having at least 80%, 85%, 90%, 95%, 96%, 97%, 98% or 99% identity thereto.

### Method for Treating and Preventing Disease and Pharmaceutical Use

The present disclosure provides a method for using, or use of, any of the aforementioned AAV capsid proteins, rAAV virions, pharmaceutical compositions and aflibercept-encoding polynucleotides of the present disclosure in treating, alleviating or ameliorating a disease or disorder.

In some embodiments, provided is a method for delivering a gene product to a subject in need thereof, comprising administering to the subject an effective amount of any of the aforementioned AAV capsid proteins, rAAV virions, pharmaceutical compositions and aflibercept-encoding polynucleotides of the present disclosure.

In some embodiments, provided is a method for delivering a gene product to a target cell, comprising contacting the target cell with any of the aforementioned AAV capsid proteins, rAAV virions, pharmaceutical compositions and aflibercept-encoding polynucleotides of the present disclosure. In some specific embodiments, the target cell is selected from the group consisting of a hepatocyte, a pancreatic cell, a skeletal muscle cell, a cardiomyocyte, a fibroblast, a retinal cell, a synovial joint cell, a lung cell, a T cell, a neuron, a neuroglial cell, a stem cell, an endothelial cell, and a cancer cell. In some specific embodiments, the target cell is *in vitro;* in some other specific embodiments, the target cell is *in vivo.* Also provided is pharmaceutical use of any of the aforementioned AAV capsid proteins, rAAV virions, pharmaceutical compositions and aflibercept-encoding polynucleotides of the present disclosure in the preparation of a medicament for delivering a heterologous nucleic acid to a target cell.

In some embodiments, provided is a method for specifically infecting retinal cells, comprising administering to an eye a prophylactically or therapeutically effective amount of any of the aforementioned AAV capsid proteins, rAAV virions, pharmaceutical compositions and aflibercept-encoding polynucleotides of the present disclosure, for example, by intravitreal injection or subretinal injection. Also provided is pharmaceutical use of any of the aforementioned AAV capsid proteins, rAAV virions, pharmaceutical compositions and aflibercept-encoding polynucleotides of the present disclosure in the preparation of a medicament for specifically infecting retinal cells.

In some specific embodiments, any of the aforementioned AAV capsid proteins, rAAV virions, pharmaceutical compositions and aflibercept-encoding polynucleotides of the present disclosure is provided for use in treating a disease or disorder of a retinal cell selected from the group consisting of a photoreceptor cell, a retinal ganglion cell, a Muller cell, a bipolar cell, an amacrine cell, a horizontal cell, and a retinal pigment epithelial cell. In some cases, the retinal cell is a photoreceptor cell, such as a rod cell or a cone cell.

In some specific embodiments, any of the aforementioned AAV capsid proteins, rAAV virions, pharmaceutical compositions and aflibercept-encoding polynucleotides of the present disclosure is provided for use in treating a disease or disorder of a retinal cell selected from the group consisting of: acute macular neuroretinopathy; Behcet's disease; choroidal neovascularization; diabetic uveitis; histoplasmosis; macular degeneration, such as acute macular degeneration, non-exudative age-related macular degeneration and exudative age-related macular degeneration; edema, such as macular edema, cystoid macular edema and diabetic macular edema; multifocal choroiditis; ocular trauma which affects a posterior ocular site or location; ocular tumors; retinal disorders, such as central retinal vein occlusion, diabetic retinopathy (including proliferative diabetic retinopathy), proliferative vitreoretinopathy (PVR), retinal arterial occlusive disease, retinal detachment, and uveitic retinal disease; sympathetic ophthalmia; Vogt Koyanagi-Harada (VKH) syndrome; uveal diffusion; a posterior ocular condition caused by or influenced by an ocular laser treatment; a posterior ocular condition caused by or influenced by a photodynamic therapy; photocoagulation, radiation retinopathy; epiretinal membrane disorders; branch retinal vein occlusion; anterior ischemic optic neuropathy; non-retinopathy diabetic retinal dysfunction; retinoschisis; retinitis pigmentosa; glaucoma; Usher syndrome, cone-rod dystrophy; Stargardt disease (fundus flavimaculatus); inherited macular degeneration; chorioretinal degeneration; Leber's congenital amaurosis; congenital stationary night blindness; choroideremia; Bardet-Biedl syndrome; macular telangiectasia; Leber's hereditary optic neuropathy; retinopathy of prematurity; and disorders of color vision, including achromatopsia, protanopia, deuteranopia, and tritanopia, and hereditary retinitis pigmentosa.

In some embodiments, provided is a method for using, or use of, any of the aforementioned AAV capsid proteins, rAAV virions, pharmaceutical compositions and aflibercept-encoding polynucleotides of the present disclosure in the treatment of eye diseases. The eye diseases include, but are not limited to: age-related macular degeneration (AMD), wet AMD, dry AMD, retinal neovascularization, choroidal neovascularization, diabetic retinopathy, proliferative diabetic retinopathy, retinal vein occlusion, central retinal vein occlusion, branch retinal vein occlusion, diabetic macular edema, diabetic retinal ischemia, ischemic retinopathy or diabetic retinal edema. Alternatively, intravitreal injection or subretinal injection is employed.

In some embodiments, provided is a method for using, or use of, any of the aforementioned AAV capsid proteins, rAAV virions, pharmaceutical compositions and aflibercept-encoding polynucleotides of the present disclosure in the treatment of retina-related diseases.

Also provided is pharmaceutical use of any of the aforementioned AAV capsid proteins, rAAV virions, pharmaceutical compositions and aflibercept-encoding polynucleotides of the present disclosure in the preparation of a medicament for treating or preventing the above diseases.

### Route of Administration

The present disclosure provides a method of using any of the aforementioned AAV capsid proteins, rAAV virions, pharmaceutical compositions and aflibercept-encoding polynucleotides of the present disclosure in the treatment of the aforementioned eye diseases (such as retina-related diseases), by intraocular injection.

In some embodiments, intraocular injection includes intravitreal injection, subretinal injection, suprachoroidal injection, or any other convenient modes or routes of administration that will result in delivery of the rAAV virion to the eye. Other convenient modes or routes of administration include, but are not limited to, intravenous, intraarterial, periocular, intracameral, subconjunctival and sub-Tenon's injection and topical administration and intranasal administration.

### BRIEF DESCRIPTION OF THE DRAWINGS

FIG. 1 shows a schematic diagram of the AAV2 capsid protein sequence and the initiation sites of VP1, VP2 and VP3, and loop domains I-V.
FIG. 2 shows a schematic diagram of the insertion of a peptide fragment between positions 587 and 588 of a variant AAV capsid protein of the present disclosure, having a mutation at position 708.
FIG. 3 shows the results of fluorescence signal detection in the retinas of mice infected with the rAAV vector viruses AAV2 seq1, AAV2 seq2, AAV2 seq3, AAV2 seq4, AAV2 seq5, AAV2 seq6 and AAV2, and AAV2.7m8 of the present disclosure.
FIG. 4A and FIG. 4B show the results of fluorescence signal detection in the retinas of mice infected with the rAAV vector viruses AAV2 seq1, AAV2 seq2, AAV2 seq3, AAV2 seq4, AAV9 seq2 and AAV2, and AAV2.7m8 of the present disclosure. FIG. 4A shows the ophthalmoscopy results of week 1. FIG. 4B shows the ophthalmoscopy results of week 4.
FIG. 5A shows a schematic diagram of the structure of a vector comprising a nucleic acid molecule that expresses VEGF Trap (aflibercept); FIG. 5B shows a schematic diagram of an AAV packaging plasmid.
FIG. 6 shows the results of testing the expression of the target gene protein (aflibercept) from the rAAV vector viruses AAV2 seq2 and AAV2.7m8 in mouse eyeballs.
FIG. 7 shows the results of testing the expression of the target gene protein (aflibercept) from the rAAV vector viruses AAV2 seq2 and AAV2.7m8 in rabbit eyeballs, which are the contents of the target gene (aflibercept) protein in the aqueous humor 14 days after virus injection.
FIG. 8 shows the results of testing the expression of the target gene protein (aflibercept) from the rAAV vector viruses AAV2 seq2 and AAV2.7m8 in rabbit eyeballs, which are the molar concentrations of the target gene protein in the aqueous humor 28 days after virus injection.

### DETAILED DESCRIPTION

To facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

"AAV" is an abbreviation for adeno-associated virus, and can be used to refer to the virus itself or derivatives thereof. Unless otherwise indicated, this encompasses all AAV subtypes as well as naturally occurring and recombinant forms. "AAV" includes, but is not limited to, AAV type 1 (AAV1), AAV type 2 (AAV2), AAV type 3 (AAV3), AAV type 4 (AAV4), AAV type 5 (AAV5), AAV type 6 (AAV6), AAV type 7 (AAV7), AAV type 8 (AAV8), AAV type 9 (AAV9), AAV type 10 (AAV10), AAVrh type 10 (AAVrh10), as well as avian AAV, bovine AAV, canine AAV, equine AAV, primate AAV, non-primate AAV and ovine AAV of different species.

AAV is a nonpathogenic parvovirus composed of a 4.7 kb single-stranded DNA genome within a non-enveloped icosahedral capsid. The genome contains three open reading frames (ORFs) flanked by inverted terminal repeats (ITRs) that function as the viral origin of replication and packaging signal. The rep ORF encodes four nonstructural proteins which play roles in viral replication, transcriptional regulation, site-specific integration and virion assembly. The cap ORF encodes three structural proteins (VP1-3) which assemble to form a 60-mer viral capsid. Finally, an ORF present as an alternate reading frame within the cap gene produces the assembly-activating protein (AAP), a viral protein that localizes AAV capsid proteins to the nucleus and functions in the capsid assembly process.

The genomic sequences of various serotypes of AAV, as well as the sequences of the native terminal repeats (ITRs), Rep proteins and capsid subunits are known in the art. Such sequences can be found in the literature or in public databases such as GenBank. See, e.g., GenBank accession numbers NC_002077.1 (AAV1), AF063497.1 (AAV1), NC_001401.2 (AAV2), AF043303.1 (AAV2), J01901.1 (AAV2), U48704.1 (AAV3), NC_001729.1 (AAV3), NC_001829.1 (AAV4), U89790.1 (AAV4), NC_006152.1 (AAV5), AF085716.1 (AAV5), AF028704.1 (AAV6), NC_006260.1 (AAV7), AF513851.1 (AAV7), AF513852.1 (AAV8), NC_006261.1 (AAV8), and AY530579.1 (AAV9). See also Srivistava et al. (1983) J. Virology 45:555; Chiorini et al. (1998) J. Virology 71:6823; Chiorini et al. (1999) J. Virology 73:1309; Bantel-Schaal et al. (1999) J. Virology 73:939; Xiao et al. (1999) J. Virology 73:3994; Muramatsu et al. (1996) Virology 221 :208; Shade et al. ,(1986) J. Virol. 58:921; Gao et al. (2002) Proc. Nat. Acad. Sci. USA 99:11854; Moris et al. (2004) Virology 33:375-383; and WO00/28061, WO99/61601, WO98/11244, US6156303, WO2012145601A, WO2017197355A and WO2018022905A. The above are incorporated in the present disclosure by reference in their entirety.

For the GH loop or loop IV of the AAV capsid, see, e.g., van Vliet et al. (2006) Mol. Ther. 14:809; Padron et al. (2005) J. Virol. 79:5047; and Shen et al. (2007) Mol. Ther. 15:1955. "AAV viral particle" or "AAV virion" refers to a viral particle composed of at least one AAV capsid protein and an encapsidated AAV polynucleotide.

"rAAV" refers to recombinant adeno-associated virus. "Recombinant" as applied to a polynucleotide means that the polynucleotide is the product of various combinations of cloning, restriction or ligation steps, and other processes that result in a construct that is different from a polynucleotide found in nature. A recombinant virus is a viral particle comprising a recombinant polynucleotide.

If an "AAV virion" comprises a heterologous polynucleotide (i.e., a polynucleotide other than a wild-type AAV genome, e.g., a gene product to be delivered to a target cell (such as a transgene or RNAi), it is typically referred to as a "recombinant AAV (rAAV) virion" or an "rAAV viral particle" or an "rAAV vector virus". In general, the heterologous polynucleotide is flanked by at least one, and generally by two AAV inverted terminal repeats (ITRs).

"rAAV vector" encompasses rAAV virions, which include rAAV polynucleotides; and also encompasses polynucleotides encoding rAAV (e.g., a single-stranded polynucleotide encoding rAAV (ss-rAAV); a double-stranded polynucleotide encoding rAAV (ds-rAAV), e.g., plasmids encoding rAAV; and the like).

"AAV variant", "AAV mutant" or "capsid-variant rAAV" refers to a viral particle composed of: (a) a variant AAV capsid protein, wherein the variant AAV capsid protein comprises at least one amino acid difference (e.g., amino acid substitution, insertion or deletion) relative to a corresponding parental AAV capsid protein, wherein the AAV capsid protein differs from or does not correspond to the amino acid sequence of a naturally occurring AAV capsid protein; and optionally, (b) comprising a heterologous polynucleotide encoding a heterologous gene product, wherein the variant AAV capsid protein confers increased binding to heparan or heparan sulfate proteoglycan compared to the binding to an AAV virion comprising a corresponding parental AAV capsid protein. "Packaging" refers to a series of intracellular events that result in the assembly and encapsidation of an AAV particle.

The "rep" and "cap" genes refer to polynucleotide sequences encoding replication and encapsidation proteins of adeno-associated viruses. AAV rep and cap are referred to herein as AAV "packaging genes".

"Helper virus" refers to a virus that allows an AAV (e.g., wild-type AAV) to be replicated and packaged by a mammalian cell. A variety of helper viruses for AAVs are known in the art, including adenoviruses, herpesviruses and poxviruses such as vaccinia. Adenoviruses encompass a number of different subgroups, although adenovirus type 5 of subgroup C is most commonly used. Viruses of the herpes family include, for example, herpes simplex viruses (HSV) and Epstein-Barr viruses (EBV), as well as cytomegaloviruses (CMV) and pseudorabies viruses (PRV). Many adenoviruses, herpesviruses and the like of human, non-human mammalian and avian origin are known and available from institutions such as the ATCC.

"Helper virus function" or "helper function" refers to a function encoded in a helper virus genome which allows AAV replication and packaging (in conjunction with other requirements for replication and packaging described herein). In the present disclosure, "helper virus function" can be provided in a number of ways, including by providing a helper virus or providing, for example, polynucleotide sequences encoding the requisite functions to a producer cell in transport.

An "infectious" virus or viral particle is one that comprises a suitably assembled viral capsid and is capable of delivering a polynucleotide component into a cell for which the viral species is tropic. The term does not necessarily imply that the virus has any replication capacity. Assays for counting infectious viral particles are well-known in the art. Viral infectivity can be expressed as the ratio of infectious viral particles to total viral particles. Methods of determining the ratio of infectious viral particles to total viral particles are known in the art. See, e.g., Grainger et al. (2005) Mol. Ther. 11:S337 (describing a TCID₅₀ infectious titer assay); and Zolotukhin et al. (1999) Gene Ther. 6:973.

"Tropism" or "specificity" refers to the preferential targeting of cells of a particular host species or a particular type of cells within a host species by a virus (e.g., an AAV). For example, a virus that can infect cells of the heart, lung, liver, and muscle has a broader (i.e., increased) tropism relative to a virus that can infect only lung and muscle cells. Tropism can also include the dependence of a virus on particular types of cell surface molecules of the host. For example, some viruses can only infect cells with surface glycosaminoglycans, while other viruses can only infect cells with sialic acid (such dependence can be tested by using various cell lines lacking particular classes of molecules as potential host cells for viral infection). In some cases, the tropism of a virus describes the relative preferences of the virus. For example, a first virus may be able to infect all cell types but is more successful in infecting those cells with surface glycosaminoglycans. A second virus can be considered to have similar (or identical) tropism to the first virus if the second virus also prefers the same characteristics (for example, the second virus is also more successful in infecting those cells with surface glycosaminoglycans), even if the absolute transduction efficiencies are not similar. For example, the second virus may be more effective than the first virus in infecting every given cell type tested, but if the relative preferences are similar (or identical), the second virus can still be considered to have similar (or identical) tropism to the first virus. In some embodiments, the tropism of a virion comprising the variant AAV capsid protein of the present disclosure is not altered relative to a naturally occurring virion. In some embodiments, the tropism of a virion comprising the variant AAV capsid protein of the present disclosure is expanded (i.e., broadened) relative to a naturally occurring virion. In some embodiments, the tropism of a virion comprising the variant AAV capsid protein of the present disclosure is reduced relative to a naturally occurring virion.

"Polynucleotide" refers to a polymeric form of nucleotides of any length, or an analog thereof, including deoxyribonucleotides or ribonucleotides. A polynucleotide may comprise modified nucleotides, such as methylated nucleotides and nucleotide analogs, and may be interrupted by non-nucleotide components. If present, modifications to the nucleotide structure may be made before or after the assembly of the polymer. Polynucleotides can refer interchangeably to double-stranded and single-stranded molecules. Unless otherwise specified, the polynucleotide of the present disclosure encompasses the double-stranded form and two complementary single-stranded forms that constitute the double-stranded form.

"Homology" or "identity" refers to sequence similarity between two polynucleotide sequences or between two polypeptides. When positions in two compared sequences are occupied by identical nucleotides or amino acid monomer subunits, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. Sequence similarity can be determined in a number of different manners. To determine sequence identity, sequences can be aligned using the methods and computer programs, including BLAST, available at the Internet website nebi.nlm.nih.gov/BLAST/. Another alignment algorithm is FASTA, which is available in the Genetics Computing Group (GCG) package, from Madison, Wisconsin, USA, a wholly owned subsidiary of Oxford Molecular Group, Inc. Other alignment techniques are described in Methods in Enzymology, vol. 266: Computer Methods for Macromolecular Sequence Analysis (1996), ed. Doolittle, Academic Press, Inc., a division of Harcourt Brace & Co., SanDiego, California, USA. Of particular interest are alignment programs that permit gaps in the sequence. The Smith-Waterman is one type of algorithm that permits gaps in sequence alignments. See Meth. Mol. Biol. 70:173-187 (1997). The GAP program using the Needleman and Wunsch alignment method can also be used to align sequences. See J. Mol. Biol. 48:443-453 (1970).

"Gene" refers to a polynucleotide comprising an open reading frame that is capable of encoding a particular gene product during transcription and sometimes after translation. "Gene" or "coding sequence" refers to an *in vitro* or *in vivo* nucleotide sequence that encodes a gene product. In some cases, a gene consists of or consists essentially of a coding sequence, i.e., a sequence that encodes a gene product. In other cases, a gene includes additional non-coding sequences. For example, a gene may or may not comprise regions preceding and following the coding region (e.g., 5' UTR, 3' UTR, and intervening sequences (introns) between individual coding segments (exons)).

"Gene product" is a molecule resulting from expression of a particular gene, e.g., a polypeptide, an aptamer, an interfering RNA, or a mRNA. In some embodiments, the "gene product" is a polypeptide, peptide, protein or interfering RNA, including short interfering RNA (siRNA), miRNA or small-hairpin RNA (shRNA). In some specific embodiments, the gene product is a therapeutic gene product, such as a therapeutic polypeptide. The therapeutic gene confers beneficial effects on the cell or tissue in which it is present, or on a mammal in which the gene is expressed. Beneficial effects include amelioration of a sign or symptom of a condition or disease, prevention or inhibition of a condition or disease, or conferral of a desired characteristic. When a gene encodes a polypeptide, "gene product" and "product expressed from the target gene" are used interchangeably.

"RNA interfering agent" or "RNAi agent" includes any agent (or polynucleotide encoding such an agent) that can be used to alter the expression of a gene (as defined above). Examples of RNAi agents known to those of ordinary skill in the art include, but are not limited to, (i) siRNA agents ("small interfering" or "short interfering RNA" (or siRNA)); (ii) antisense RNA; (iii) CRISPR agents; (iv) zinc finger nuclease agents; and (v) transcription activator-like effector nuclease (TALEN) agents. The definitions of (i)-(v) in WO2017197355A are incorporated herein by reference in their entirety.

siRNA agents are RNA duplexes that target nucleotides of a gene of interest ("target gene"). "RNA duplex" refers to the structure formed by the complementary pairing between two regions of an RNA molecule, thereby forming a region of double stranded RNA (dsRNA). siRNA is "targeted" to a gene in that the nucleotide sequence of the duplex portion of siRNA is complementary to the nucleotide sequence of the target gene.

In some embodiments, the length of the duplex of siRNA is less than 30 nucleotides. In some embodiments, the duplex may be 29, 28, 27, 26, 25, 24, 23, 22, 21, 20, 19, 18, 17, 16, 15, 14, 13, 12, 11 or 10 nucleotides in length. In some embodiments, the duplex is 19-25 nucleotides in length. The RNA duplex portion of siRNA can be part of a hairpin structure. Hairpin-containing siRNA agents may also be referred to as "shRNA (short-hairpin RNA) agents". In addition to the duplex portion, the hairpin structure may contain a loop portion located between the two sequences that form the duplex. The loop may vary in length. In some embodiments, the loop is 5, 6, 7, 8, 9, 10, 11, 12 or 13 nucleotides in length. The hairpin structure may also contain 3' or 5' overhang portions. In some embodiments, the overhang is a 3' or 5' overhang that is 0, 1, 2, 3, 4 or 5 nucleotides in length. In general, the level of expression product (e.g., a mRNA or polypeptide) of a target gene is reduced by a siRNA agent (e.g., a siRNA or shRNA) that contains specific double-stranded nucleotide sequences that are complementary to at least a 19-25 nucleotide long segment (e.g., a sequence of 20-21 nucleotides) of the target gene transcript, including the 5' untranslated (UTR) region, the ORF, or the 3' UTR region. In some embodiments, the short interfering RNA is about 19-25 nt in length. See, e.g., PCT applications WO0/44895, WO99/32619, WO01/75164, WO01/92513, WO01/29058, WO01/89304, WO02/16620, and WO02/29858; and U.S. patent publication No. 20040023390 (description of siRNA technology). The siRNA and/or shRNA may be encoded by a nucleic acid sequence, and the nucleic acid sequence can also include a promoter. The nucleic acid sequence may also include a polyadenylation signal. In some embodiments, the polyadenylation signal is a synthetic minimal polyadenylation signal. The antisense RNA is an RNA that is complementary to a gene expression product. For example, an antisense RNA targeting a specific mRNA is an RNA-based agent (or may be a modified RNA) that is complementary to the mRNA, wherein hybridization of the antisense RNA to the mRNA alters the expression of the mRNA (e.g., by altering the stability of the RNA, altering the translation of the RNA, and the like). Nucleic acids encoding an antisense RNA are also included in "antisense RNA".

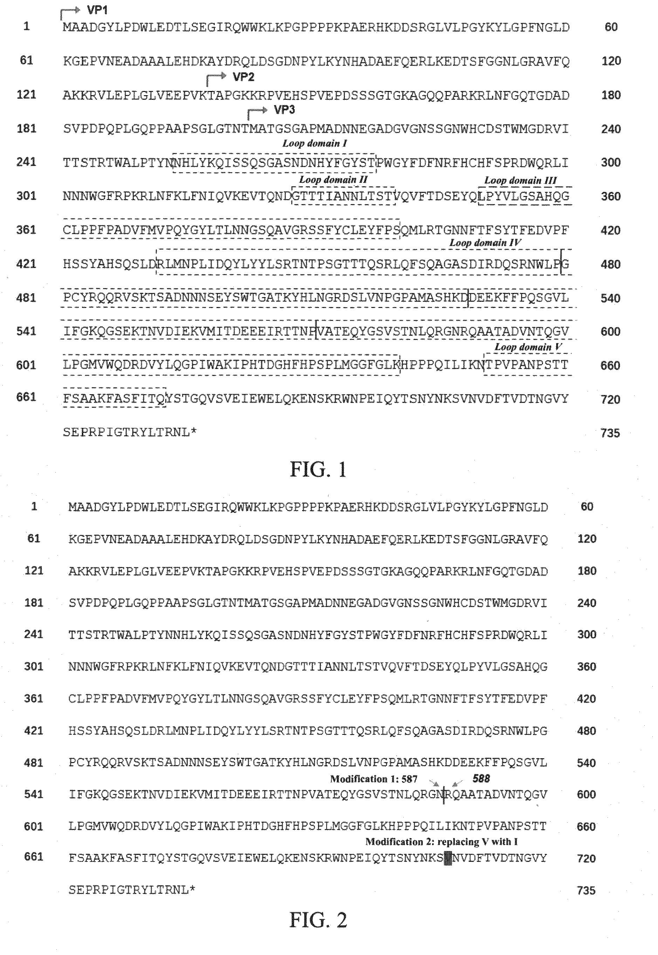

"VEGF" refers to a vascular endothelial growth factor that induces angiogenesis or an angiogenic process. The term includes various subtypes of VEGF (also known as vascular permeability factor (VPF) and VEGF-A) (see FIGs. 2(A) and (B) of U.S. patent application publication No. 20120100136) that are produced by, for example, alternative splicing of the VEGF-A/VPF gene, including VEGF121, VEGF165 and VEGF189. Further, "VEGF" includes VEGF-related angiogenic factors such as PIGF (placenta growth factor), VEGF-B, VEGF-C, VEGF-D and VEGF-E, which act through a cognate VEFG receptor (i.e., VEGFR) to induce angiogenesis or an angiogenic process.

"sFlt-1" or "sFlt-1 protein" refers herein to a polypeptide sequence or a functional fragment thereof that is at least 90% or more homologous to the naturally occurring human sFLT-1 sequence, such that the sFlt-1 protein or polypeptide binds to VEGF and/or a VEGF receptor.

"Polypeptide", "peptide" and "protein" refer to polymers of amino acids of any length.

The terms also encompass modified amino acid polymers, for example, disulfide bond formation, glycosylation, lipidation, phosphorylation or conjugation with a labeling component.

"Regulatory element" or "regulatory sequence" is a nucleotide sequence involved in an interaction of molecules that contributes to the functional regulation of a polynucleotide, including replication, duplication, transcription, splicing, translation or degradation of the polynucleotide. The regulation may affect the frequency, speed or specificity of the process, and may be enhancing or inhibitory in nature. Known control elements include, for example, transcriptional regulatory sequences such as promoters and enhancers. A promoter is a DNA region capable of binding to RNA polymerase and initiating transcription of a coding region usually located downstream (in the 3' direction) of the promoter under certain conditions.

"Expression vector" is a vector comprising a region encoding a target gene, and is used for achieving the expression of the gene product in an intended target cell. The vector includes a polynucleotide encoding the gene product of interest. An expression vector also comprises control elements operatively linked to the encoding region to facilitate the expression of the gene product in the target. The combination of control elements, such as promoters, enhancers, UTRs and miRNA targeting sequences, and one or more genes to which they are operably linked for expression is sometimes referred to as an "expression cassette". Many expression cassettes are known and available in the art, or can be readily constructed from components available in the art.

"Operatively linked" or "operably linked" refers to a juxtaposition of genetic elements, wherein the elements are in a relationship permitting them to operate in an intended manner. For example, a promoter is operatively linked to a coding polynucleotide sequence if the promoter helps initiate the transcription of the coding polynucleotide sequence. There may be intervening nucleic acid residues between the promoter and the coding polynucleotide sequence so long as this functional relationship is maintained.

"Administering" or "introducing" refers to delivering a vector for recombinant gene or protein expression to a cell, to cells and/or organs of a subject, or to a subject. Such administering or introducing may take place *in vivo, in vitro* or *ex vivo.* A vector for expressing a gene product can be introduced into a cell by transfection, which typically means inserting heterologous DNA into a cell by physical means (e.g., calcium phosphate transfection, electroporation, microinjection or lipofection); infection, which typically refers to introduction by an infectious agent; or transduction, which typically means stably infecting a cell with a cell or transferring genetic material from one microorganism to another by a viral agent (e.g., a bacteriophage). "Transformation" is typically used to refer to bacteria comprising heterologous DNA or cells that express an oncogene and have been switched to a continuous growth mode, such as tumor cells. A vector used to "transform" a cell may be a plasmid, virus or other vehicle. A cell is generally referred to as "transduced", "infected", "transfected" or "transformed" according to the means used for administering, introducing or inserting heterologous DNA (i.e., the vector) into the cell. "Transduce", "transfect" and "transform" are used interchangeably herein regardless of the method of introducing heterologous DNA.

"Host cell" refers to a cell which has been transduced, infected, transfected or transformed with a vector. The term encompasses the original transduced, infected, transfected or transformed cell and progeny thereof. A vector may be a plasmid, a viral particle, a phage, or the like. Culture conditions such as temperature and pH are apparent to those skilled in the art.

"Treatment" is generally used to mean obtaining a desired pharmacological and/or physiological effect. The effect may be prophylactic in terms of completely or partially preventing a disease or a symptom thereof, e.g., reducing the possibility that the disease or symptom thereof occurs in the subject, and/or may be therapeutic in terms of partially or completely curing a disease and/or an adverse effect arising from the disease. "Treatment" covers any treatment of a disease in a mammal, and includes: (a) preventing the disease from occurring in a subject who may be predisposed to the disease but has not yet been diagnosed as having it; (b) inhibiting or arresting the progression of the disease; or (c) relieving the disease (or a symptom arising therefrom) or causing regression of the disease. The therapeutic agent may be administered before, during or after the onset of the disease or injury. The treatment of ongoing disease, where the treatment stabilizes or reduces the undesirable clinical symptoms of the patient, is of particular interest. Some preferred protocols are to perform such treatment prior to complete loss of function in the affected tissue. Some preferred protocols are to administer the treatment of the present disclosure during the symptomatic stage of the disease and, in some cases, after the symptomatic stage of the disease.

"Effective amount" comprises an amount sufficient to ameliorate or prevent a symptom or sign of a medical condition. An effective amount also refers to an amount sufficient to allow or facilitate diagnosis. The effective amount for a particular subject may vary depending on factors such as the condition to be treated, the general health of the subject, the method and dose of administration, and the severity of side effects. An effective amount may be the maximum dose or administration regimen to avoid significant side effects or toxic effects.

"Retinal cell" in the present disclosure may refer to any one of the cell types that comprise the retina, such as retinal ganglion cells; amacrine cells; horizontal cells; bipolar cells; photoreceptor cells including rod and cone cells; Müller glial cells; astrocytes (such as retinal astrocytes); and retinal pigment epithelial cells.

"Individual", "subject" and "patient" are used interchangeably herein, and include, but are not limited to, humans and non-human primates, e.g., simians, humans and other mammalian animals (such as horses, sheep, goats, dogs, cats and rodents (such as mice and rats)); humans are preferred.

In the present disclosure, "polypeptide" and "protein" are used interchangeably.

### Examples

The present disclosure is further described below with reference to examples, which, however, are not intended to limit the present disclosure. Experimental procedures without conditions specified in the examples of the present disclosure are generally conducted according to conventional conditions, or according to conditions recommended by the manufacturers of the starting materials or commercial products. If the source of a reagent is not shown, the reagent is obtained from any molecular biology reagent supplier in quality/purity for molecular biology applications.

### Example 1. AAV Capsid Engineering

Molecular dynamics simulation and protein fingerprinting are widely applied to protein engineering and antibody affinity modification. Through dynamics simulations of the physicochemical properties of antibodies and receptors, the effect of amino acid alterations on local affinities can be predicted to some extent.

The heparan sulfate receptor has been shown to be the primary receptor responsible for mediating AAV cell infection, and also plays an important role in the infection of retinal and optic nerve cells. Research confirms that in the AAV2 natural serotype capsid protein amino acid sequence, according to its spatial structural characteristics, there are 5 loop domains (as shown in FIG. 1) that are associated with the infection ability of the virus. The insertion of a short peptide of 7-12 amino acids between positions 587 and 588 of loop domain IV does not affect virus capsid protein assembly, and can greatly change the way the AAV capsid interacts with the cell receptor (heparan sulfate), thereby affecting the ability of an AAV to infect the host cell.

The present disclosure utilizes our independently developed bioinformatics algorithm to insert several random amino acid sequences (7-12 amino acids in length) between positions 587 and 588 of the AAV2 natural serotype capsid protein amino acid sequence and to predict the interaction between the capsid protein and the heparan sulfate receptor. A sequence predicted by the algorithm to be able to greatly improve the infection ability was selected for subsequent verification. Based on the algorithmic predictions, the 6 amino acid sequences seq1 to seq6 as shown in Table 1 were selected. Each of the sequences is 10 amino acids in length. Meanwhile, the amino acid at position 708 of the AAV2 natural serotype capsid protein amino acid sequence valine (V) was substituted with isoleucine (I). The insertion between positions 587 and 588 and the amino acid substitution at position 708 are shown in FIG. 2. An engineered AAV capsid protein sequence was obtained. Meanwhile, a capsid was obtained by inserting seq2 at a corresponding position ofAAV9.

**Table 1. The amino acid sequences inserted between positions 587 and 588 of the AAV capsid and their corresponding numbers**

| Numbering of inserted polypeptides and their amino acid sequences | | Corresponding AAV capsid numbers and their amino acid sequence numbers | |
|---|---|---|---|
| seq1 | LALAETTRPA (SEQ ID NO: 17) | AAV2 seq1 | SEQ ID NO: 3 |
| seq2 | LALGDTTRPA (SEQ ID NO: 18) | AAV2 seq2 | SEQ ID NO: 4 |
| seq3 | LALGETTRNA (SEQ ID NO: 19) | AAV2 seq3 | SEQ ID NO: 5 |
| seq4 | LAKADTTKNA (SEQ ID NO: 20) | AAV2 seq4 | SEQ ID NO: 6 |
| seq5 | LAKDDTTRNA (SEQ ID NO: 21) | AAV2 seq5 | SEQ ID NO: 7 |
| seq6 | LALADTTKNA (SEQ ID NO: 22) | AAV2 seq6 | SEQ ID NO: 8 |
| seq2 | LALGDTTRPA (SEQ ID NO: 18) | AAV9 seq2 | SEQ ID NO: 9 |

| | | | |
|---|---|---|---|
| (Note: Linkers are underlined.) | | | |

Since AAV capsid proteins are formed by assembling the three monomer proteins VP1, VP2 and VP3 in certain ratios after expression, they have a one level higher structure than a common recombinant protein. Therefore, the functional effect of the polypeptide insertions occurring in AAV capsid proteins is not limited to the changes they effected in physicochemical properties. They are more likely to have more complicated effects in the process of assembling the three monomer proteins VP1, VP2 and VP3.

### Example 2. Tests of rAAV Vector Viruses for Abilities to Infect Retina of Mouse Model

To verify the function, we separately used capsids having the 8 sequences SEQ ID NO: 3 to SEQ ID NO: 8, AAV2 (SEQ ID NO: 1) and AAV2.7m8 (SEQ ID NO: 2) to package the plasmid pGOI, which contains and can express the EGFP target gene driven by the CMV promoter. The above AAV vectors packaged with different AAV capsids and having the ability to express EGFP were injected into the vitreous humor of mice at the same dose. EGFP expression was detected by fundus fluorescence imaging. The differences in intensity between EGFP fluorescence signals were used to assess the differences in efficiency with which different AAV capsids infected retinal tissue.

The packaging and purification steps of the rAAV vector viruses comprise: transfecting 293T adherent cells with a triple-plasmid system of pHelper, pR2C9 and pGOI; collecting cells and the supernatant, performing purification by iodixanol centrifugation, then performing ultrafiltration and concentration, and replacing the buffer with DPBS. After the packaging and purification of the viruses were complete, titers (measured in vg) were determined by qPCR. The 8 viruses expressing the EGFP target gene (their capsids were AAV2, AAV2.7m8, AAV2 seq1, AAV2 seq2, AAV2 seq3, AAV2 seq4, AAV2 seq5, and AAV2 seq6) were diluted with a DPBS buffer to the same titer of 1.03E+12. Ten-week-old C57 mice (purchased from Beijing Vital River Laboratory Technology Co., Ltd.) were administered unilateral-eye intravitreal injections of 1 µL. Each AAV vector was injected into three mice (n = 3). During week 1 to week 4 after injection, fundus fluorescence imaging was performed every week.

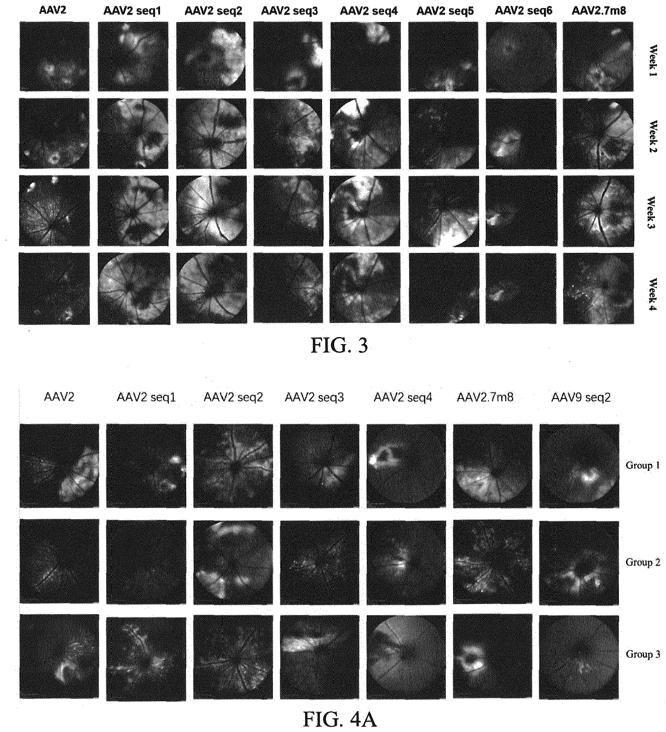

Some results are shown in FIG. 3. The AAV2 group, the AAV2 seq5 group and the AAV2 seq6 group showed significantly lower fluorescence signal intensity than the other groups. The AAV2 seq1 group, the AAV2 seq2 group, the AAV2 seq3 group and the AAV2 seq4 group showed similar fluorescence signal intensity to AAV2.7m8, which indicates that the efficiency of infecting retinal tissue is relatively high.

The "LALGDTTRPA" sequence in Table 1 was inserted at the same position (between positions 588 and 589) of the AAV9 capsid to obtain AAV9 seq2. The 7 capsids AAV2, AAV2.7m8, AAV2 seq1, AAV2 seq2, AAV2 seq3, AAV2 seq4 and AAV9 seq2 were separately used to package a plasmid vector, which contains and can express the EGFP target gene driven by the CMV promoter. The above AAV vectors packaged with different AAV capsids and having the ability to express EGFP were injected into the vitreous humor of mice at the same dose. EGFP expression was detected by fundus fluorescence imaging. The differences in intensity between EGFP signals were used to assess the differences in efficiency with which different AAV capsids infected retinal tissue.

The AAV viruses were subjected to vector packaging and purification using the methods described above. The 7 viruses expressing the EGFP target gene (their capsids were AAV2, AAV2.7m8, AAV2 seq1, AAV2 seq2, AAV2 seq3, AAV2 seq4, AAV2 seq5, and AAV9 seq2) were diluted with a DPBS buffer to the same titer of 1.03E+12. Ten-week-old C57 mice were administered unilateral-eye intravitreal injections of 1 µL. Each AAV vector was injected into three mice (n = 3). During week 1 to week 4 after injection, fundus fluorescence imaging was performed every week.

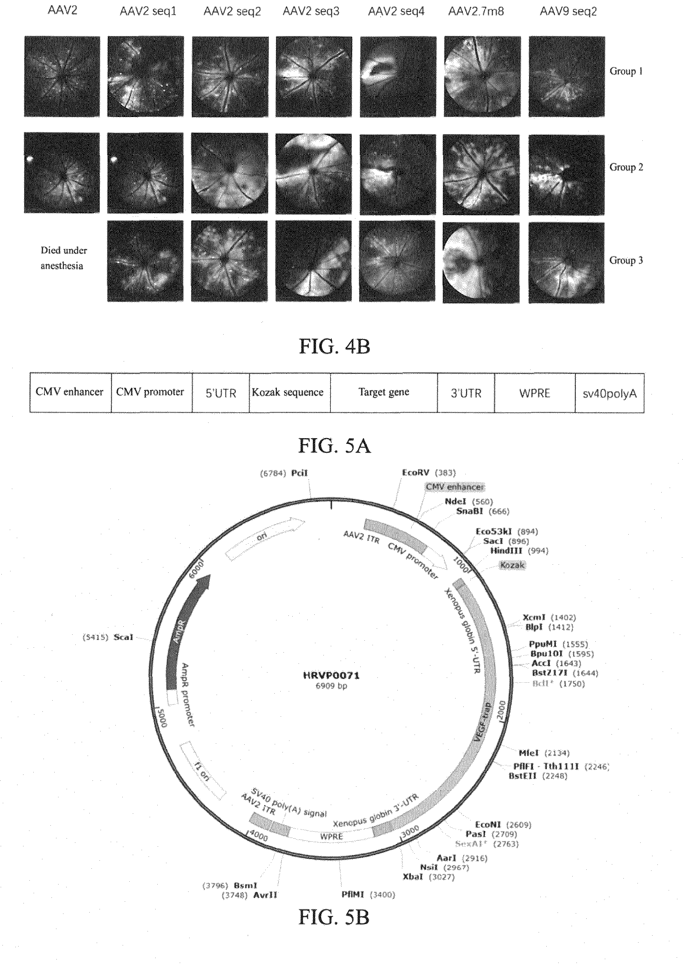

The results are shown in FIG. 4A and FIG. 4B. The AAV2 group, the AAV2 seq2 group and the AAV2 seq3 group showed significantly higher fluorescence signal intensity than the other groups and showed similar intensity to AAV2.7m8.

### Example 3. Tests of rAAV Vector Viruses for Biological Function of Infecting Retina of Mouse Model

To further verify the ability of the seq2 capsid to infect retinal tissue and its related biological functions, AAV2 seq2 and AAV2.7m8 were separately used to package a target plasmid vector containing aflibercept driven by the CMV promoter (AAV2 seq2-aflibercept and AAV2.7mβ-aflibercept). The two viruses were intravitreally injected into New Zealand rabbits and C57 mice. Two weeks after injection, the aflibercept content in injected eye tissue was determined and used to assess the infection of the retina by corresponding AAV viruses and their related biological functions.

The structure of the gene expression cassette the rAAV delivered comprises, from the 5' end to the 3' end (as shown in FIG. 5A): a CMV enhancer, a promoter, a 5' UTR, a Kozak sequence (gccacc), the VEGF inhibitor aflibercept, a 3' UTR, WPRE and SV40polyA. The base sequence of the CMV enhancer is set forth in SEQ ID NO: 28. The base sequence of the CMV promoter is set forth in SEQ ID NO: 29. The 5' UTR is set forth in SEQ ID NO: 30. The Kozak sequence is set forth in SEQ ID NO: 31. The 3' UTR is set forth in SEQ ID NO: 32. WPRE is set forth in SEQ ID NO: 33. SV40polyA is set forth in SEQ ID NO: 34.

Construction of expression vector: An expression cassette was constructed. An AAV packaging plasmid expressing aflibercept was constructed by conventional molecular biology operations such as enzyme digestion, ligation, transformation, clone screening and identification, which sequentially comprises: a CMV enhancer, a promoter, a 5' UTR, a Kozak sequence (gccacc), a VEGF trap (aflibercept), a 3' UTR, WPRE and SV40polyA. The expression cassette was flanked by inverted terminal repeats (ITRs). A schematic diagram of its structure is shown in FIG. 5B, where EcoRV and BSMI are restriction enzyme cutting sites.

Vector packaging and purification of rAAV vector viruses were performed according to the method in Example 2.

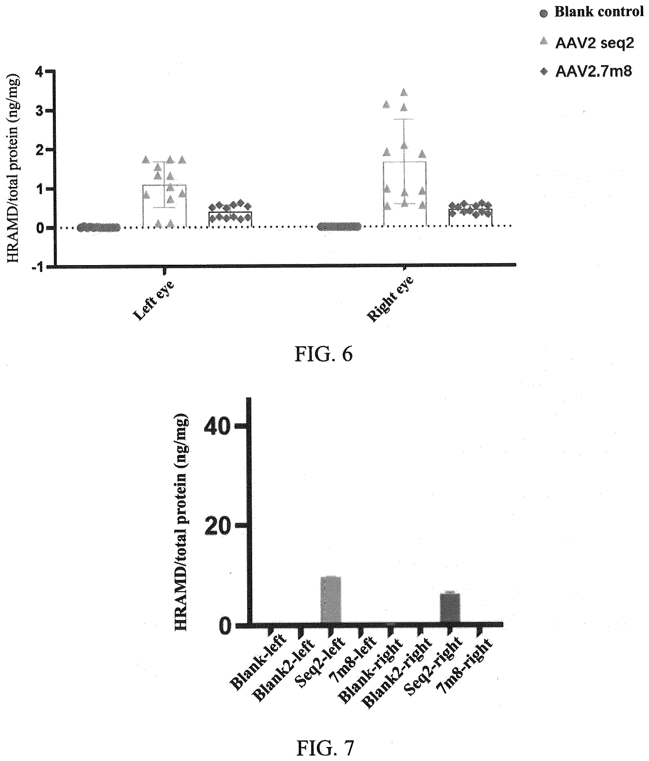

The two purified viruses AAV seq2-aflibercept and AAV2.7m8-aflibercept were diluted with a DPBS buffer to the same titer of 9E+12 vg/mL. Eight-to-ten-week-old C57 mice were administered bilateral-eye intravitreal injections of 1 µL. Each rAAV vector virus was injected into four mice (n = 4). Two weeks after injection, the mice were sacrificed, and eye tissue was collected and ground. Of each injection group, samples from the left eyes and samples from the right eyes were ground using two different methods, respectively. Method 1 for the left eyes: Samples were ground with PBS + protease inhibitor and centrifuged, and the supernatants were then collected. Method 2 for the right eyes: Samples were ground with RIP Buffer + protease inhibitor and centrifuged, and the supernatants were then collected. The supernatants were assayed for aflibercept protein content using the enzyme-linked immunoassay (4 samples per injection group, each sample assayed in triplicate). The results are shown in FIG. 6. The blank control is a PBS injection group, and the ordinate HRAMD/total protein refers to the amount (measured in ng) of aflibercept protein in 1 mg of tissue total protein.

**Table 2. The expression levels of the target gene protein (aflibercept) in mouse eye tissue**

| Tissue | Blank control | AAV2 seq2 | AAV2.7m8 |
|---|---|---|---|
| Left eye | 0.01±0.01 | 1.10±0.59 | 0.39±0.17 |
| Right eye | 0 | 1.66±1.08 | 0.44±0.11 |

The results in FIG. 6 and Table 2 show that the AAV seq2-aflibercept group expressed more than the AAV2.7m8-aflibercept group in mice.

### Example 4. Tests of rAAV Vector Viruses for Biological Function of Infecting Retina of Rabbit Model

Vector packaging and purification of rAAV vector viruses were performed according to the method in Example 2.

The two purified viruses AAV seq2-aflibercept and AAV2.7m8-aflibercept were diluted with a DPBS buffer to 4E+9 vg/mL. Eight-to-ten-week-old New Zealand rabbits (purchased from Beijing Vital River Laboratory Technology Co., Ltd.) were administered bilateral-eye intravitreal injections of 50 µL. Each rAAV vector virus was injected into two rabbits (n = 2). Two weeks after injection, the rabbits were sacrificed, and the aqueous humor, the vitreous humor and whole-eye tissue were collected and ground. Of each injection group, samples from the left eyes and samples from the right eyes were ground using two different methods, respectively. Method 1 for the left eyes: Samples were ground with PBS + protease inhibitor and centrifuged, and the supernatants were then collected. Method 2 for the right eyes: Samples were ground with RIP Buffer + protease inhibitor and centrifuged, and the supernatants were then collected. The supernatants were assayed for aflibercept protein content using the enzyme-linked immunoassay (2 samples per injection group, each sample assayed in triplicate). The results are shown in FIG. 7. The blank control is a PBS injection group, and the ordinate HRAMD/total protein refers to the amount (measured in ng) of aflibercept protein in 1 mg of tissue total protein. The results in FIG. 7 show that the AAV seq2-aflibercept group expressed more than the AAV2.7m8-aflibercept group in New Zealand rabbits. Due to the large size of rabbit eyes, aflibercept was diluted in tissue fluid after whole eye grinding. Therefore, its concentration was much lower than those in the vitreous humor and the aqueous humor. Since AAV-packaged vectors successfully express the carried target gene protein only after successful infection of cells by AAV viruses, the results in FIG. 7 indicate that the AAV2 seq2 capsid infects cells with higher efficiency than the AAV2.7m8 capsid. Moreover, we also compared the expression levels of the protein of the target gene (VEGF inhibitor) the AAV2 seq2 and AAV2.7m8 capsids had delivered. Method: Eight New Zealand rabbits were administered bilateral-eye injections according to the doses shown in Table 3. Each dose group contained 2 rabbits and hence 4 eyes. A total of 4 groups are designed. On day 28 after injection, the animals were sacrificed, and the aqueous humor was extracted and assayed by ELISA for the molar concentration of the target gene protein.

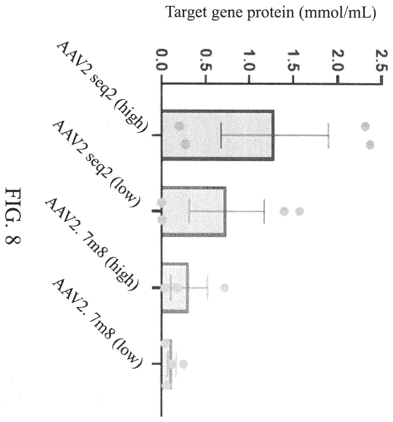

The results are shown in FIG. 8. When injected at the same dose, AAV2 seq2 led to a significantly higher molar concentration of the expressed target gene protein than AAV2.7m8.

**Table 3.**

| Group | Viral titer | Injection volume | vg/eye |
|---|---|---|---|
| AAV2 seq2 (high) | 6E12 vg/mL | 100µL | 6E11 vg/eye |
| AAV2 seq2 (low) | 2E12 vg/mL | 100µL | 2E11 vg/eye |
| AAV2.7m8 (high) | 6E12 vg/mL | 100µL | 6E11 vg/eye |
| AAV2.7m8 (low) | 2E12 vg/mL | 100µL | 2E11 vg/eye |

Some sequences of the present disclosure are shown below:
> Amino acid sequence of AAV2 capsid 





 









SEQ ID NO: 1
> Amino acid sequence of AAV2.7m8 capsid 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALGETTRPARQAATADVNTQGVLPGMVWQDRDVYLQGPIWAKIPHTDGH
FHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL
SEQ ID NO: 2
> Amino acid sequence of AAV2 seq1 capsid 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALAETTRPARQAATADVNTQGVLPGMVWQDRDVYLQGPIWAKIPHTDGH
 
FHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL
SEQ ID NO: 3
> Amino acid sequence of AAV2 seq2 capsid 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALGDTTRPARQAATADVNTQGVLPGMVWQDRDVYLQGPIWAKIPHTDGH
FHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL*
SEQ ID NO: 4
> Amino acid sequence of AAV2 seq3 capsid 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALGETTRNARQAATADVNTQGVLPGMVWQDRDVYLQGPIWAKIPHTDGH
FHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL
SEQ ID NO: 5
> Amino acid sequence of AAV2 seq4 capsid MAADGYLPDWLEDTLSEGIRQWWKLKPGPPPPKPAERHKDDSRGLVLPGYKY 
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLAKADTTKNARQAATADVNTQGVLPGMVWQDRDVYLQGPIWAKIPHTDG
HFHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL
SEQ ID NO: 6
> Amino acid sequence of AAV2 seq5 capsid 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLAKDDTTRNARQAATADVNTQGVLPGMVWQDRDVYLQGPIWAKIPHTDG
HFHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL
SEQ ID NO: 7
> Amino acid sequence of AAV2 seq6 capsid 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
 
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALADTTKNARQAATADVNTQGVLPGMVWQDRDVYLQGPIWAKIPHTDG
HFHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL
SEQ ID NO: 8
> Amino acid sequence of AAV9 seq2 capsid 
MAADGYLPDWLEDNLSEGIREWWALKPGAPQPKANQQHQDNARGLVLPGYK
YLGPGNGLDKGEPVNAADAAALEHDKAYDQQLKAGDNPYLKYNHADAEFQE
RLKEDTSFGGNLGRAVFQAKKRLLEPLGLVEEAAKTAPGKKRPVEQSPQEPDSS
AGIGKSGAQPAKKRLNFGQTGDTESVPDPQPIGEPPAAPSGVGSLTMASGGGAP
VADNNEGADGVGSSSGNWHCDSQWLGDRVITTSTRTWALPTYNNHLYKQISNS
TSGGSSNDNAYFGYSTPWGYFDFNRFHCHFSPRDWQRLINNNWGFRPKRLNFK
LFNIQVKEVTDNNGVKTIANNLTSTVQVFTDSDYQLPYVLGSAHEGCLPPFPAD
VFMIPQYGYLTLNDGSQAVGRSSFYCLEYFPSQMLRTGNNFQFSYEFENVPFHSS
YAHSQSLDRLMNPLIDQYLYYLSKTINGSGQNQQTLKFSVAGPSNMAVQGRNYI
PGPSYRQQRVSTTVTQNNNSEFAWPGASSWALNGRNSLMNPGPAMASHKEGED
RFFPLSGSLIFGKQGTGRDNVDADKVMITNEEEIKTTNPVATESYGQVATNHQSA
QLALGDTTRPAAQAQTGWVQNQGILPGMVWQDRDVYLQGPIWAKIPHTDGNF
HPSPLMGGFGMKHPPPQILIKNTPVPADPPTAFNKDKLNSFITQYSTGQVSVEIE
WELQKENSKRWNPEIQYTSNYYKSNNVEFAVNTEGVYSEPRPIGTRYLTRNL
SEQ ID NO: 9
> Amino acid sequence of EGFP 
MVSKGEELFTGVVPILVELDGDVNGHKFSVSGEGEGDATYGKLTLKFICTTGKL
PVPWPTLVTTLTYGVQCFSRYPDHMKQHDFFKSAMPEGYVQERTIFFKDDGNY
KTRAEVKFEGDTLVNRIELKGIDFKEDGNILGHKLEYNYNSHNVYIMADKQKN
GIKVNFKIRHNIEDGSVQLADHYQQNTPIGDGPVLLPDNHYLSTQSALSKDPNE
KRDHMVLLEFVTAAGITLGMDELYK
SEQ ID NO: 10
> seq1 (without linker)
   LAETTRP
   SEQ ID NO: 11
> seq2 (without linker)
   LGDTTRP
   SEQ ID NO: 12
> seq3 (without linker)
   LGETTRN
   SEQ ID NO: 13
> seq4 (without linker)
   KADTTKN
   SEQ ID NO: 14
> seq5 (without linker)
   KDDTTRN
   SEQ ID NO: 15
> seq6 (without linker)
   LADTTKN
   SEQ ID NO: 16
> seq1
   LALAETTRPA
   SEQ ID NO: 17
> seq2
   LALGDTTRPA
   SEQ ID NO: 18
> seq3
   LALGETTRNA
   SEQ ID NO: 19
> seq4
   LAKADTTKNA
   SEQ ID NO: 20
> seq5
   LAKDDTTRNA
   SEQ ID NO: 21
> seq6
   LALADTTKNA
   SEQ ID NO: 22
> Amino acid sequence of AAV2 seq2 capsid (excluding V708I) 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALGDTTRPARQAATADVNTQGVLPGMVWQDRDVYLQGPIWAKIPHTDGH
FHPSPLMGGFGLKHPPPQILIKNTPVPANPSTTFSAAKFASFITQYSTGQVSVEIE
 
WELQKENSKRWNPEIQYTSNYNKSVNVDFTVDTNGVYSEPRPIGTRYLTRNL
SEQ ID NO: 23
> Amino acid sequence of AAV2 seq3 capsid (excluding V708I) 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALGETTRNARQAATADVNTQGVLPGMVWQDRDVYLQGPIWAKIPHTDGH
FHPSPLMGGFGLKHPPPQILIKNTPVPANPSTTFSAAKFASFITQYSTGQVSVEIE
WELQKENSKRWNPEIQYTSNYNKSVNVDFTVDTNGVYSEPRPIGTRYLTRNL
SEQ ID NO: 24
> Amino acid sequence of AAV2 seq4 capsid (excluding V708I) 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLAKADTTKNARQAATADVNTQGVLPGMVWQDRDVYLQGPIWAKIPHTDG
HFHPSPLMGGFGLKHPPPQILIKNTPVPANPSTTFSAAKFASFITQYSTGQVSVEIE
WELQKENSKRWNPEIQYTSNYNKSVNVDFTVDTNGVYSEPRPIGTRYLTRNL
SEQ ID NO: 25
> Amino acid sequence of AAV2 seq5 capsid (excluding V708I) 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
 
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLAKDDTTRNARQAATADVNTQGVLPGMVWQDRDVYLQGPIWAKIPHTDG
HFHPSPLMGGFGLKHPPPQILIKNTPVPANPSTTFSAAKFASFITQYSTGQVSVEIE
WELQKENSKRWNPEIQYTSNYNKSVNVDFTVDTNGVYSEPRPIGTRYLTRNL
SEQ ID NO: 26
> Amino acid sequence of AAV2 seq6 capsid (excluding V708I) 
MAADGYLPDWLEDTLSEGIRQWWKLKPGPPPPKPAERHKDDSRGLVLPGYKY
LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALADTTKNARQAATADVNTQGVLPGMVWQDRDVYLQGPIWAKIPHTDG
HFHPSPLMGGFGLKHPPPQILIKNTPVPANPSTTFSAAKFASFITQYSTGQVSVEIE
WELQKENSKRWNPEIQYTSNYNKSVNVDFTVDTNGVYSEPRPIGTRYLTRNL
SEQ ID NO: 27
> Sequence of CMV enhancer 
CGTTACATAACTTACGGTAAATGGCCCGCCTGGCTGACCGCCCAACGACCCC
CGCCCATTGACGTCAATAATGACGTATGTTCCCATAGTAACGCCAATAGGGAC
TTTCCATTGACGTCAATGGGTGGAGTATTTACGGTAAACTGCCCACTTGGCAG
TACATCAAGTGTATCATATGCCAAGTACGCCCCCTATTGACGTCAATGACGGT
AAATGGCCCGCCTGGCATTATGCCCAGTACATGACCTTATGGGACTTTCCTAC
TTGGCAGTACATCTACGTATTAGTCATCGCTATTACCATG
SEQ ID NO: 28
>Sequence of CMV promoter 
TGCTGATGCGGTTTTGGCAGTACACCAATGGGCGTGGATAGCGGTTTGACTC
ACGGGGATTTCCAAGTCTCCACCCCATTGACGTCAATGGGAGTTTGTTTTGG
CACCAAAATCAACGGGACTTTCCAAAATGTCGTAATAACCCCGCCCCGTTGA
CGCAAATGGGCGGTAGGCGTGTACGGTGGGAGGTCTATATAAGCAGAGCTCG
TTTAGTGAACCGTCAGATCGCCTGGAGAGGCCATCCACGCTGTTTTGACCTC
 
CATAGTGGACACCGGGACCGATCCAGCCTCCGCGTCTCAGGGGAAGCTT
SEQ ID NO: 29
>Sequence of 5' UTR
   CTTGTTCTTTTTGCAGAAGCTCAGAATAAACGCTCAACTTTGG
   SEQ ID NO: 30
> Kozak sequence
   GCCACC
   SEQ ID NO: 31
> Sequence of 3' UTR 
GTCTAGAAACCAGCCTCAAGAACACCCGAATGGAGTCTCTAAGCTACATAAT
ACCAACTTACACTTTACAAAATGTTGTCCCCCAAAATGTAGCCATTCGTATCT
GCTCCTAATAAAAAGAAAGTTTCTTCAC
SEQ ID NO: 32
> Sequence of WPRE 
AATCAACCTCTGGATTACAAAATTTGTGAAAGATTGACTGGTATTCTTAACTA
TGTTGCTCCTTTTACGCTATGTGGATACGCTGCTTTAATGCCTTTGTATCATGCT
ATTGCTTCCCGTATGGCTTTCATTTTCTCCTCCTTGTATAAATCCTGGTTGCTGT
CTCTTTATGAGGAGTTGTGGCCCGTTGTCAGGCAACGTGGCGTGGTGTGCAC
TGTGTTTGCTGACGCAACCCCCACTGGTTGGGGCATTGCCACCACCTGTCAG
CTCCTTTCCGGGACTTTCGCTTTCCCCCTCCCTATTGCCACGGCGGAACTCAT
CGCCGCCTGCCTTGCCCGCTGCTGGACAGGGGCTCGGCTGTTGGGCACTGAC
AATTCCGTGGTGTTGTCGGGGAAATCATCGTCCTTTCCTTGGCTGCTCGCCTG
TGTTGCCACCTGGATTCTGCGCGGGACGTCCTTCTGCTACGTCCCTTCGGCCC
TCAATCCAGCGGACCTTCCTTCCCGCGGCCTGCTGCCGGCTCTGCGGCCTCT
TCCGCGTCTTCGCCTTCGCCCTCAGACGAGTCGGATCTCCCTTTGGGCCGCCT
CCCCGC
SEQ ID NO: 33
> Sequence of SV40polyA: 
CCTAGGTAAGATACATTGATGAGTTTGGACAAACCACAACTAGAATGCAGTG
AAAAAAATGCTTTATTTGTGAAATTTGTGATGCTATTGCTTTATTTGTAACCAT
TATAAGCTGCAATAAACAAGTTATCGATAGATCT
SEQ ID NO: 34
> Amino acid sequence of AAV9 capsid 
MAADGYLPDWLEDNLSEGIREWWALKPGAPQPKANQQHQDNARGLVLPGYK
YLGPGNGLDKGEPVNAADAAALEHDKAYDQQLKAGDNPYLKYNHADAEFQE
RLKEDTSFGGNLGRAVFQAKKRLLEPLGLVEEAAKTAPGKKRPVEQSPQEPDSS
AGIGKSGAQPAKKRLNFGQTGDTESVPDPQPIGEPPAAPSGVGSLTMASGGGAP
VADNNEGADGVGSSSGNWHCDSQWLGDRVITTSTRTWALPTYNNHLYKQISNS
TSGGSSNDNAYFGYSTPWGYFDFNRFHCHFSPRDWQRLINNNWGFRPKRLNFK
LFNIQVKEVTDNNGVKTIANNLTSTVQVFTDSDYQLPYVLGSAHEGCLPPFPAD
VFMIPQYGYLTLNDGSQAVGRSSFYCLEYFPSQMLRTGNNFQFSYEFENVPFHSS
 
YAHSQSLDRLMNPLIDQYLYYLSKTINGSGQNQQTLKFSVAGPSNMAVQGRNYI
PGPSYRQQRVSTTVTQNNNSEFAWPGASSWALNGRNSLMNPGPAMASHKEGED
RFFPLSGSLIFGKQGTGRDNVDADKVMITNEEEIKTTNPVATESYGQVATNHQSA
QAQAQTGWVQNQGILPGMVWQDRDVYLQGPIWAKIPHTDGNFHPSPLMGGFG
MKHPPPQILIKNTPVPADPPTAFNKDKLNSFITQYSTGQVSVEIEWELQKENSKR
WNPEIQYTSNYYKSNNVEFAVNTEGVYSEPRPIGTRYLTRNL
SEQ ID NO: 37
> Sequence of aflibercept protein 
MPLLLLLPLLWAGALASDTGRPFVEMYSEIPEIIHMTEGRELVIPCRVTSPNITVT
LKKFPLDTLIPDGKRIIWDSRKGFIISNATYKEIGLLTCEATVNGHLYKTNYLTHR
QTNTIIDVVLSPSHGIELSVGEKLVLNCTARTELNVGIDFNWEYPSSKHQHKKLV
NRDLKTQSGSEMKKFLSTLTIDGVTRSDQGLYTCAASSGLMTKKNSTFVRVHEK
DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNH
YTQKSLSLSPGK
SEQ ID NO: 38
> Nucleic acid sequence 1 of aflibercept 
ATGCCCCTGCTCCTGCTCCTGCCCCTGCTGTGGGCCGGCGCCCTGGCTAGCG
ACACCGGCAGACCCTTCGTGGAGATGTACAGCGAGATCCCCGAGATCATCCA
CATGACCGAGGGCAGAGAGCTGGTGATCCCCTGCAGAGTGACAAGCCCCAA
CATCACCGTGACCCTGAAAAAATTCCCTCTGGACACCCTGATCCCCGACGGC
AAGAGAATCATCTGGGACAGCAGAAAGGGCTTCATCATCAGCAACGCCACC
TACAAGGAGATCGGCCTGCTGACCTGCGAGGCCACCGTGAACGGCCACCTG
TACAAGACCAACTACCTGACCCACAGACAGACCAACACCATCATCGACGTG
GTGCTGAGCCCTAGCCACGGCATCGAGCTGAGCGTGGGCGAGAAGCTGGTG
CTGAACTGCACCGCTAGAACCGAGCTGAACGTGGGCATCGACTTCAACTGG
GAGTACCCTAGCAGCAAGCATCAGCACAAGAAGCTGGTGAACAGAGACCTG
AAGACACAGAGCGGCAGCGAGATGAAGAAGTTCCTGAGCACCCTGACCATC
GACGGCGTGACAAGAAGCGACCAAGGCCTGTACACCTGCGCCGCTAGCAGC
GGCCTGATGACCAAGAAGAACAGCACCTTCGTGAGAGTGCATGAGAAGGAC
AAGACCCACACCTGCCCCCCCTGCCCCGCTCCTGAACTGCTGGGCGGCCCTA
GCGTGTTCCTGTTCCCCCCCAAGCCCAAGGACACACTCATGATCAGCAGAAC
CCCCGAGGTGACCTGCGTGGTCGTGGACGTGAGCCACGAGGACCCCGAGGT
GAAGTTCAACTGGTACGTGGACGGCGTGGAGGTGCACAACGCCAAGACCAA
GCCTAGAGAGGAGCAGTACAACAGCACCTACAGAGTGGTGAGCGTGCTGAC
CGTGCTGCACCAAGACTGGCTGAACGGCAAGGAGTACAAGTGCAAGGTGAG
CAACAAGGCCCTGCCCGCCCCCATCGAGAAGACCATCAGCAAGGCCAAGGG
GCAGCCTAGAGAGCCCCAAGTGTACACCCTGCCCCCTAGCAGAGACGAGCT
GACCAAGAACCAAGTGAGCCTGACCTGCCTGGTGAAGGGCTTCTACCCTAG
 
CGACATCGCCGTGGAGTGGGAGAGCAACGGGCAGCCCGAGAACAACTACAA
GACCACCCCCCCCGTGCTGGACAGCGACGGCAGCTTCTTCCTGTACAGCAAG
CTGACCGTGGACAAGAGCAGATGGCAGCAAGGCAACGTGTTCAGCTGCAGC
GTGATGCACGAGGCCCTGCACAACCACTACACACAGAAGAGCCTGAGCCTG
AGCCCCGGCAAGTGA
SEQ ID NO: 39
> Nucleic acid sequence 2 of aflibercept 
ATGCCTCTGCTCCTGCTGCTCCCTCTGCTGTGGGCCGGCGCCCTGGCCAGCG
ACACAGGCCGCCCCTTCGTGGAAATGTACAGCGAGATCCCTGAGATCATCCA
CATGACAGAGGGCCGGGAACTGGTCATCCCTTGTAGAGTGACATCTCCAAAC
ATCACCGTGACCCTGAAAAAGTTCCCCCTGGACACACTGATCCCTGACGGCA
AAAGAATCATCTGGGATTCCCGGAAGGGCTTCATCATCAGCAATGCCACCTA
CAAGGAAATCGGACTGCTGACCTGTGAAGCCACAGTGAACGGCCACCTGTA
CAAGACCAACTACCTGACCCATAGGCAGACCAATACCATCATCGATGTGGTG
CTGTCTCCTTCTCACGGCATCGAGCTCAGCGTCGGCGAGAAACTGGTGCTGA
ACTGCACCGCCAGAACCGAGCTGAACGTGGGCATCGACTTCAACTGGGAAT
ACCCCAGTTCTAAGCACCAGCACAAGAAACTGGTTAATAGAGATCTGAAGAC
CCAAAGCGGCAGCGAGATGAAAAAGTTTCTGTCTACACTGACAATTGACGGT
GTTACCAGAAGCGATCAGGGCCTGTATACATGCGCCGCTTCTAGCGGCCTGAT
GACCAAGAAGAACAGCACCTTCGTGCGGGTGCACGAGAAGGACAAGACCC
ACACCTGCCCCCCCTGTCCAGCTCCTGAACTGCTGGGAGGCCCTAGCGTGTT
CCTGTTCCCTCCAAAGCCTAAGGACACCCTTATGATTAGCAGAACACCTGAG
GTGACCTGCGTGGTGGTCGACGTGTCCCACGAGGACCCCGAGGTGAAATTTA
ACTGGTACGTGGATGGCGTGGAAGTTCACAACGCCAAGACCAAGCCCAGAG
AGGAACAGTACAACAGCACATATAGAGTGGTGTCCGTGCTGACCGTTCTGCA
CCAGGACTGGCTGAATGGAAAAGAGTACAAGTGCAAGGTGTCCAACAAGGC
CCTGCCTGCCCCTATCGAGAAGACTATCAGCAAGGCTAAGGGCCAACCTCGG
GAACCCCAGGTGTACACCCTGCCCCCCAGCCGGGACGAGCTGACAAAGAAC
CAGGTGTCCCTGACATGCCTGGTGAAGGGATTTTACCCTAGCGATATCGCCGT
GGAATGGGAGAGCAACGGCCAGCCTGAGAACAACTACAAAACGACCCCTCC
TGTGCTGGACTCTGATGGCAGCTTCTTCCTGTACAGCAAGCTGACCGTGGAC
AAGAGCAGATGGCAGCAGGGCAACGTGTTCTCATGCAGCGTGATGCACGAG
GCCCTGCATAATCACTACACCCAGAAAAGCCTGAGCCTGAGCCCTGGCAAGT
GA
SEQ ID NO: 40
> Nucleic acid sequence 3 of aflibercept

ATGCCACTGCTCTTGCTTTTGCCCTTGCTTTGGGCTGGTGCCCTTGCCTCAGA
CACTGGAAGGCCGTTCGTAGAAATGTACTCCGAGATCCCTGAAATAATCCAC
ATGACGGAGGGGCGAGAGTTGGTGATACCATGTCGAGTTACGAGCCCGAATA
TTACAGTAACATTGAAGAAATTCCCACTCGACACTCTCATTCCAGATGGTAAG
AGAATTATATGGGATTCACGAAAGGGCTTCATTATATCTAACGCTACTTACAAA

GAGATAGGCTTGCTCACATGCGAGGCAACTGTCAATGGCCACTTGTATAAAA
CCAATTATCTCACACATAGGCAGACTAATACGATAATAGACGTTGTCCTTTCTC
CGAGCCATGGCATTGAATTGAGTGTCGGTGAGAAGCTCGTATTGAACTGCAC
TGCTAGGACCGAGCTTAACGTAGGGATCGACTTTAACTGGGAGTACCCATCAT
CAAAGCATCAGCATAAAAAGCTCGTCAACCGGGACCTTAAGACGCAGAGTG
GCTCAGAGATGAAGAAGTTCCTTTCTACACTGACCATCGATGGTGTAACCAG
AAGTGACCAGGGACTCTATACCTGTGCCGCATCCTCCGGCCTTATGACGAAG
AAAAACAGCACATTTGTTCGGGTCCACGAGAAGGACAAGACTCATACGTGT
CCGCCGTGTCCAGCCCCAGAATTGCTCGGGGGGCCTTCCGTGTTTCTGTTCC
CTCCAAAGCCAAAGGACACGCTTATGATTTCAAGGACTCCCGAAGTCACCTG
CGTCGTGGTTGACGTTTCTCATGAGGACCCAGAGGTGAAATTCAACTGGTAC
GTGGATGGAGTAGAGGTACATAACGCCAAAACAAAACCACGAGAAGAGCAG
TATAACTCAACGTATCGGGTAGTTTCTGTGCTTACGGTTCTCCACCAAGATTG
GTTGAATGGAAAAGAGTATAAGTGTAAAGTCTCAAACAAAGCCCTGCCGGC
ACCCATCGAGAAGACCATTAGCAAAGCAAAAGGCCAACCTAGAGAACCGCA
GGTGTACACGTTGCCACCGTCAAGGGATGAACTTACGAAGAATCAAGTATCA
CTCACATGTTTGGTTAAAGGATTTTATCCCAGCGACATCGCCGTAGAATGGGA
GAGTAACGGCCAGCCTGAAAATAACTATAAGACGACACCCCCTGTTTTGGAT
TCTGACGGTTCATTTTTTTTGTATAGTAAACTCACAGTCGACAAGAGTCGCTG
GCAACAAGGTAATGTATTTAGCTGTAGCGTGATGCATGAAGCTTTGCACAACC
ACTATACACAGAAGTCCTTGTCTCTGTCTCCGGGAAAATGA
SEQ ID NO: 41

## Claims

1. A variant adeno-associated virus (AAV) capsid protein, comprising an inserted polypeptide relative to a parental AAV capsid protein, wherein the inserted polypeptide comprises a polypeptide selected from any one of 1)-6) or any combination thereof:
1) LGDTTRP (SEQ ID NO: 12) or LALGDTTRPA (SEQ ID NO: 18);
2) LAETTRP (SEQ ID NO: 11) or LALAETTRPA (SEQ ID NO: 17);
3) LGETTRN (SEQ ID NO: 13) or LALGETTRNA (SEQ ID NO: 19);
4) KADTTKN (SEQ ID NO: 14) or LAKADTTKNA (SEQ ID NO: 20);
5) KDDTTRN (SEQ ID NO: 15) or LAKDDTTRNA (SEQ ID NO: 21); and
6) LADTTKN (SEQ ID NO: 16) or LALADTTKNA (SEQ ID NO: 22).

2. The variant AAV capsid protein according to claim 1, wherein the polypeptide of any one of 1)-6) or any combination thereof is located in a GH loop of the AAV capsid protein; preferably, follows any one of the amino acid residues between positions 570 to 611 of a VP1 encoded amino acid sequence of a parental AAV2 capsid protein;
and more preferably, is located between positions 587 and 588 of the VP1 encoded amino acid sequence of the parental AAV2 capsid protein, or between positions 588 and 589 of a VP1 encoded amino acid sequence of a parental AAV9 capsid protein, or at a corresponding position of other parental AAV serotype capsid proteins.

3. The variant AAV capsid protein according to claim 1 or 2, further comprising an amino acid residue point mutation selected from one or more of 1L, 15P, 34A, 57D, 66K, 81Q, 101R, 109T, 144K, 144M, 164K, 176P, 188I, 196Y, 226E, 236V, 240T, 250S, 312K, 363L, 368H, 449D, 456K, 463Y, 472N, 484C, 524T, 535S, 551S, 593E, 698V, 708I, 719M, 721L and 735Q, wherein the mutation is at a position based on an AAV2 capsid protein set forth in SEQ ID NO: 1, or a corresponding position of other AAV serotype capsid proteins;
preferably, the point mutation is 708I.

4. The variant AAV capsid protein according to any one of claims 1-3, having an amino acid sequence that is set forth in any one of SEQ ID NOs: 3-9 and 23-27 or has at least 90% or 95% sequence identity thereto.

5. A recombinant adeno-associated virus (rAAV) virion, comprising:
(a) the variant AAV capsid protein according to any one of claims 1-4, and
(b) a heterologous polynucleotide.

6. The rAAV virion according to claim 5, wherein the heterologous polynucleotide comprises a polynucleotide that encodes a gene product selected from the group consisting of a polypeptide, an interfering RNA and an aptamer;
the polypeptide is preferably an antibody or an antigen-binding fragment thereof or a fusion protein.

7. The rAAV virion according to claim 6, wherein the heterologous polynucleotide further comprises a polynucleotide selected from one or more of (a) to (g) below:
(a) a 5' inverted terminal repeat (5' ITR) and/or a 3' inverted terminal repeat (3' ITR),
(b) a 5' untranslated region (5' UTR) and/or a 3' untranslated region (3' UTR),
(c) a promoter,
(d) an enhancer,
(e) a post-transcriptional regulatory element,
(f) a polyadenylation signal (polyA), and
(g) a Kozak sequence;
preferably, the heterologous polynucleotide further comprises a polynucleotide selected from one or more of (a) to (g) below:
(a) a 5' ITR and/or a 3' ITR derived from AAV2,
(b) a 5' UTR and/or a 3' UTR derived from Xenopus globin,
(c) a CMV promoter,
(d) a CMV enhancer,
(e) WPRE,
(f) SV40 polyA, and
(g) a Kozak sequence.

8. The rAAV virion according to claim 7, wherein the heterologous polynucleotide comprises, from the 5' end to the 3' end, the following polynucleotides operably linked and arranged in the order of (a) to (h) below:
(a) an enhancer,
(b) a promoter,
(c) a 5' UTR,
(d) a Kozak sequence,
(e) a polynucleotide encoding a gene product,
(f) a 3' UTR,
(g) WPRE, and
(h) a polyA.

9. The rAAV virion according to any one of claims 6-8, wherein the polypeptide is a neuroprotective polypeptide, an anti-angiogenic polypeptide, a polypeptide that enhances retinal cell function;
preferably, the anti-angiogenic polypeptide is a VEGF antagonist;
more preferably, the VEGF antagonist is aflibercept.

10. The rAAV virion according to any one of claims 5-9, wherein the heterologous polynucleotide comprises a polynucleotide encoding aflibercept set forth in SEQ ID NO: 38;
preferably, the heterologous polynucleotide comprises a polynucleotide that is set forth in any one of SEQ ID NOs: 39-41 or has at least 90% or 95% sequence identity thereto.

11. An isolated polynucleotide, encoding the variant AAV capsid protein according to any one of claims 1-4.

12. A vector, comprising:
(a) the isolated polynucleotide according to claim 11, or
(b) the isolated polynucleotide according to claim 11 and the heterologous polynucleotide according to any one of claims 5-10.

13. A host cell, comprising the vector according to claim 12.

14. A pharmaceutical composition, comprising:
(a) the rAAV virion according to any one of claims 5-10; and
(b) one or more pharmaceutically acceptable carriers, diluents, excipients or buffers.

15. A method for specifically infecting a retinal cell, wherein the method comprises a step of intraocularly, preferably intravitreally or subretinally, injecting an effective amount of the rAAV virion according to any one of claims 5-10, the heterologous polynucleotide according to any one of claims 5-10, or the pharmaceutical composition according to claim 14.

16. A method for treating an eye-related disease, comprising administering to a subject in need thereof a prophylactically or therapeutically effective amount of the rAAV virion according to any one of claims 5-10, the heterologous polynucleotide according to any one of claims 5-10, or the pharmaceutical composition according to claim 14, preferably by intravitreal injection or subretinal injection.

17. The method according to claim 16, wherein the eye-related disease is a retinal cell disease; preferably, the retinal cell disease is selected from the group consisting of diseases of photoreceptor cells, retinal ganglion cells, Muller cells, bipolar cells, amacrine cells, horizontal cells, and retinal pigment epithelial cells.

18. The method according to claim 16 or 17, wherein the eye-related disease is selected from the group consisting of retinitis pigmentosa, macular degeneration, wet AMD, dry AMD, retinal neovascularization, choroidal neovascularization, diabetic retinopathy, proliferative diabetic retinopathy, retinal vein occlusion, central retinal vein occlusion, branch retinal vein occlusion, diabetic macular edema, diabetic retinal ischemia, ischemic retinopathy, diabetic retinal edema, retinoschisis, glaucoma, Leber's congenital amaurosis, and/or achromatopsia.

19. A method for delivering a heterologous polynucleotide to a target cell *in vitro* and/or *in vivo,* comprising contacting the target cell with the rAAV virion according to any one of claims 5-10, the heterologous polynucleotide according to any one of claims 5-10, or the pharmaceutical composition according to claim 14.

20. An rAAV virion production system, comprising:
(a) the isolated polynucleotide according to claim 11;
(b) a heterologous polynucleotide encoding a gene product; and
(c) a helper element, having sufficient AAV rep function and helper function to package the heterologous polynucleotide encoding a gene product of (b) into the variant AAV capsid of (a).

21. A method for producing or preparing the rAAV virion according to any one of claims 5-10, comprising: introducing the vector according to claim 12 and a vector comprising a helper element into a producer cell, and packaging and purifying the rAAV virion.
